# EUROPEAN PATENT SPECIFICATION

(11) **EP 0 655 500 B1**
(45) Date of publication and mention of the grant of the patent: **12.05.1999**
(21) Application number: 94308117.4
(22) Date of filing: 03.11.1994
(51) Int. Cl.: C12N 15/29, C07K 14/415, A61K 39/36

(54) **Allergenic polypeptide from Japanese cedar pollen and dna encoding it**
Allergenisches Polypeptid von japanischen Zederpollen und diese kodierende DNA
Polypeptide alergénique provenant du pollen du cedre Japonais et DNA encodant celui-ci

(30) Priority: 05.11.1993 JP 299151/93; 20.12.1993 JP 344596/93; 27.12.1993 JP 346814/93
(43) Date of publication of application: 31.05.1995
(73) Proprietor: KABUSHIKI KAISHA HAYASHIBARA SEIBUTSU KAGAKU KENKYUJO, Okayama-shi Okayama (JP)
(72) Inventor: Namba, Motoshi, Akaiwa-gun, Okayama (JP); Torigoe, Kakuji, Kurashiki-shi, Okayama (JP); Kurimoto, Masashi, Okayama-shi, Okayama (JP)
(74) Representative: Daniels, Jeffrey Nicholas

(56) References cited:
- EP-A- 0 308 147
- WO-A-94/11512
- ALLERGY, vol.45, no.4, May 1990 pages 309 - 312 SAKAGUCHI ET AL. 'Identification of the second major allergen of Japanese cedar pollen'
- GENE., vol.40, 1985, AMSTERDAM NL pages 183 - 190 AMANN AND BROSIUS ''ATG vectors' for regulated gigh-level expression of cloned genes in Escherichia coli'
- FEBS LETTERS., vol.353, no.1, 10 October 1994, AMSTERDAM NL pages 124 - 128 NAMBA ET AL. 'Molecular cloning of the second major allergen, Cry jII, from Japanese cedar pollen'
- BIOCHEMICAL AND BIOPHYSICAL RESEARCH COMMUNICATIONS, vol.201, no.2, 15 June 1994, DULUTH, MINNESOTA US pages 1021 - 1028 KOMIYAMA ET AL. 'cDNA cloning and expression of Cry j II, the second major allergen of Japanese cedar pollen'
- Pharmacia Cataloque 1997

## Description

This invention relates to a novel polypeptide causative of pollen allergy, as well as to a DNA coding the same.

In Japan for these ten and several years, the number of persons who complain of rhinitis and/or conjunctival hyperemia due to pollen allergy has steadily increased. As mass media have frequently taken up, with viewpoint of public health, this must be now one of unnegligible problems because patients are so many and incidence is in early spring where various events are held one after another.

Pollen allergy is a type of allergic disease and the antigenic substances in cedar pollen, i.e. cedar pollen allergens, are said to be one of the major causes. When cedar pollens which float in the air come into human body, then immunoglobulin E antibodies against those are immediately produced. If further cedar pollen comes into the body under these conditions, then it arises an immunoreaction with the immunoglobulin E antibodies which may arise allergic symptoms.

It is known that there are at least two distinct types of allergens in cedar pollens, which differ in antigenicity. One type is such as reported by Yasueda et al. in Journal of Allergy and Clinical Immunology, Vol.71, No.1, pp.77-86 (1983), which is now designated as "Cry j I". The other type is such as reported in Tani-ai et al., FEBS Letters, Vol.239, No.2, pp.329-332 (1988) and Sakaguchi et al., Allergy, No.45, pp.309-312 (1990), which is now designated as "Cry j II". It is said that in cedar pollens Cry j I and Cry j II are usually present at a ratio of about 50:1-5:1, as well as that sera from patients with pollen allergy generally react with both Cry j I and Cry j II. Sawatani et al. reported in Allergy, Vol.42, No.6, pp.738-747 (1993) that Cry j II exhibited approximately the same antigenicity in the intradermal and RAST tests as Cry j I did.

As described above, several types of cedar pollen allergens have been already isolated and characterized to some degree, thus it has become possible to treat and prevent pollen allergy in human by administering purified cedar pollen allergens thereto for desensitization. Recently several proposals therefor have been made: For example, Japan Patent Kokai Nos.156,926/89 and 93,730/91 propose that allergens with an amino acid sequence starting at N-terminal as represented by Asp-Asn-Pro-Ile-Asp-Ser or Ala-Ile-Asn-Ile-Phe-Asn are co-valently bound to pullulan, a type of polysaccharide, and the resultant conjugates are administered as desensitizer to human. Although diagnosis and desensitization of allergic diseases generally need a large amount of purified allergens, allergens in cedar pollens are trace and very labile, thus one will encounter great difficulties if he or she uses cedar pollen as sole source for diagnostic and desensitizing agents in pollen allergy.

While recent recombinant DNA technology has notable advances. Nowadays, if the amino acid sequence of an objective polypeptide is not fully determined, such polypeptide can be quite easily obtained in desired amount by making a recombinant DNA which contains a DNA coding the polypeptide, and culturing a transformant which is obtainable by introducing the recombinant DNA into a microorganism or cell of animal or plant, provided that such gene is isolated and sequenced.

Because of this situation, it has been in long expectation that all the genes which code particular allergens in cedar pollen are isolated and sequenced as soon as possible. The International Patent Publication No.1,213/93, which was recently published, discloses a nucleic acid sequence for Cry j I, a type of cedar pollen allergen. However, as mentioned above, Cry j I is not a sole allergen which causes pollen allergy, therefore in order for accurate diagnosis and effective desensitization it must be one of urgent themes in the art to isolate and sequence all the genes which code other allergens.

In view of the foregoing, one object of this invention is to provide a novel polypeptide which is causative of pollen allergy.

Another object of this invention is to provide a DNA which codes such polypeptide.

Still another object of this invention is to provide a replicable recombinant DNA which contains such DNA.

Still another object of this invention to provide a transformant where such recombinant DNA has been introduced in an appropriate host.

Still another object of this invention is to provide a process to produce such polypeptide wherein recombinant DNA technology is applied.

Still another object of this invention is to provide a use of such polypeptide as desensitizer.

This invention solves the first object with a polypeptide bearing the following physicochemical properties:
(1) Molecular weight
   55,000±5,000 daltons as determined by SDS-polyacrylamide gel electrophoresis
(2) Isoelectric point
   9.0±0.5 as determined by isoelectric point electrophoresis
(3) N-Terminal amino acid sequence
   Bearing at N-terminal an amino acid sequence as represented by Ala-Met-Lys-Phe-Ile-Ala-Pro-Met-Ala-Phe-Val-Ala-Met-Gln-Leu-Ile-Ile-Met-Ala-.
(4) Ultraviolet absorption spectrum
   Showing an absorption maximum around 280nm.
(5) Solubility in solvents
   Soluble in water, physiological saline and phosphate buffer.
(6) Biological activity
   Combining with immunoglobulin E antibodies collected from bloods of patients with pollen allergy.
   Causative of pollen allergy.
(7) Stability
   Inactivated when incubated in aqueous solution (pH7.2) at 100°C for 10 minutes.
   Substantially not inactivated even when allowed to stand in aqueous solution (pH7.2) at 4°C for 1 month.

This invention solves the second object with a DNA which codes such polypeptide.

This invention solves the third object with a replicable recombinant DNA comprising such DNA and an expression vector.

This invention solves the fourth object with a transformant comprising such replicable recombinant DNA and an appropriate host to which such recombinant DNA is introduceable.

This invention solves the fifth object with a process which comprises cultivating in a culture medium a transformant which contains a DNA coding such polypeptide, and collecting the produced polypeptide from the resultant culture.

This invention solves the sixth object with a desensitizer which contains as effective ingredient such peptide.

The polypeptide of this invention is a novel substance with distinct physicochemical properties which are not seen in conventional cedar pollen allergens.

The DNA of this invention expresses the production of the polypeptide when inserted in appropriate expression vectors and then introduced in recombinant form into hosts which are not inherently able to produce the polypeptide but easily proliferatable.

The recombinant DNA of this invention expresses the production of the polypeptide when introduced into hosts which are not inherently able to produce the polypeptide but easily proliferatable.

The transformant of this invention produces the polypeptide when cultivated.

The process of this invention enables the production of such polypeptide in desired amounts using recombinant DNA technology.

The desensitizer of this invention exhibits a desensitizing effect in mammals including human when administered thereto.

The invention will now be described in further detail, by way of example only, with reference to the accompanying drawings, in which:-

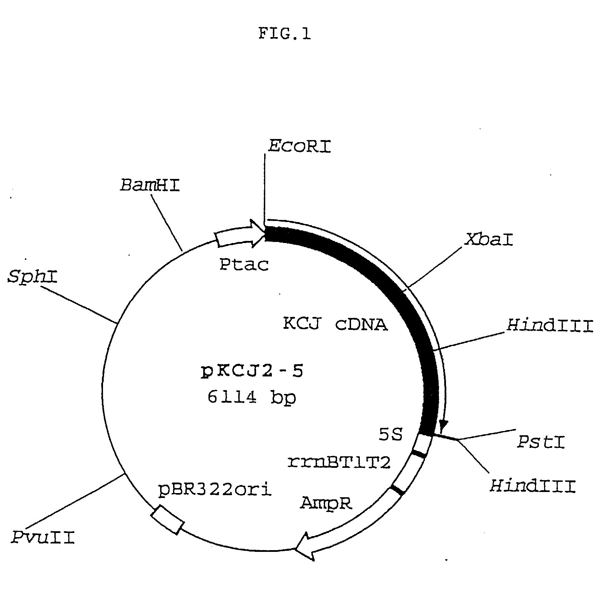

FIG.1 shows the structure of a recombinant DNA "pKCJ2-5" according to this invention.

In FIG.1 KJC cDNA designates a DNA which codes the polypeptide of this invention; Ptac, Tac promoter; rrnBT1T2, transcription terminating area for ribosome RNA operon; AmpR, ampicillin resistant gene; and ori, replication initiating point in Escherichia coli.

Now explaining this invention in conjunction with several Experiments and Examples, this invention relates to a novel polypeptide causative of pollen allergy. During our studies on allergens in cedar pollen, we found therein a hitherto unknown, entirely novel allergen. After isolating this allergen by combination of various purification methods including column chromatographies and studying its properties and nature, it was found that its nature was a polypeptide with the following physicochemical properties:
(1) Molecular weight
   40,000±5,000 daltons as determined by SDS-polyacrylamide gel electrophoresis
(2) Isoelectric point
   9.5±0.5 as determined by isoelectric point electrophoresis
(3) Partial amino acid sequence
   Bearing partial amino acid sequences as represented by
(4) Ultraviolet absorption spectrum
   Exhibiting an absorption maximum around 280nm.
(5) Solubility in solvents
   Soluble in water, physiological saline and phosphate buffer.
(6) Biological activity
   Causative of pollen allergy.
(7) Stability
   Inactivated when heated in aqueous solution (pH7.2) at 100°C for 10 minutes.
   Substantially not inactivated even when allowed to stand in aqueous solution (pH7.2) at 4°C for one month.

The polypeptide, which bears such physicochemical properties, is unknown and deemed to be a novel substance.

We energetically screened genes which might code this polypeptide, resulting in successful isolation of mRNA from cedar pollen. We applied reverse transcriptase reaction to this mRNA as template, subjected the resultant cDNA to gene amplification by the PCR method and sequenced the resultant DNA fragments, revealing that the objective gene contained a nucleic acid sequence starting at 5'-terminal as shown in the Sequence Listing with SEQ ID No.2. Then we decoded this nucleic acid sequence, revealing that as shown in the Sequence Listing with SEQ ID No.1 the polypeptide was composed of 514 amino acids at maximum. A series of experiment to determine the nucleic acid and amino acid sequences in the Sequence Listing with SEQ ID Nos.1 and 2 will be explained hereinafter.

### Experiment 1

### Purification of polypeptide

One part by weight of pollen from male flowers of cedar produced in Akita, Japan was added in about 16 parts by weight of 0.125M aqueous sodium hydrogen carbonate solution (pH8.2), extracted at 4°C for 1 hour while stirring and centrifuged, followed by collecting the supernatant. The residue was further treated similarly as above and a newly formed supernatant was pooled with the previous supernatant and added with ammonium sulfate to give 80% saturation to effect salting out. The resultant sediment was dialyzed against 50mM Tris-HCl buffer (pH7.8) for 10 hours, filtered with membrane and applied to DEAE-Sephadex column which had been equilibrated with 50mM Tris-HCl buffer (pH7.8), followed by collecting non-adsorbed fractions.

After adjusting to pH5.0 by the addition of acetic acid, the fractions were loaded to CM-Sephadex column equilibrated with 10mM acetate buffer (pH5.0) which was then applied at first with 10mM acetate buffer (pH5.0) for washing, then with an eluent comprising 0.1M phosphate buffer (pH7.0) and 0.3M sodium chloride to elute peptide components. Fractions which contained the polypeptide were collected, further loaded to Mono-S column equilibrated with 10mM phosphate buffer (pH5.0) which was then applied with 10mM Tris-HCl buffer (pH7.0) under a concentration gradient of sodium chloride increasing from 0M to 0.5M, thus the polypeptide was eluted at a sodium chloride concentration of 0.4M.

The purified polypeptide obtained in this way notably reacted with human immunoglobulin E antibodies from patients with pollen allergy, as well as with mouse anti-cedar pollen allergen antibodies. The yield was about 0.006% against the starting cedar pollen, on dry solid basis.

### Experiment 2

### Physicochemical properties of polypeptide

In this Experiment, the purified polypeptide obtained in Experiment 1 was investigated for physicochemical properties.

### Experiment 2-1

### Molecular weight

After SDS-polyacrylamide gel electrophoresis of the purified polypeptide in accordance with the method reported in U.K. Leammli, Nature, Vol.227, pp.680-685 (1970), a band was observed at a position corresponding to a molecular weight of 40,000±5,000 daltons. The molecular markers as used were calf serum albumin (67,000 daltons), obalbumin (45,000 daltons), carbonic anhydrolase (30,000 daltons), chymotrypsinogen A (25,000 daltons) and cytochrome c (12,000 daltons).

Thereafter the component of 40,000±5,000 daltons was transferred from the gel to nitrocellulose membrane and exposed to both mouse anti-cedar pollen allergen antibody and goat anti-mouse immunoglobulin antibody which had been labelled with horseradish peroxidase, resulting in a notable immunopigmentation which suggested that the component was a type of cedar pollen allergen.

### Experiment 2-2

### Isoelectric point

After measuring with isoelectric point electrophoresis, the purified polypeptide showed an isoelectric point at 9.5±0.5.

### Experiment 2-3

### Partial amino acid sequence

The purified polypeptide was trypsinized and the digest was separated by high-performance liquid chromatography using "Model Hi-Pore PR-318", a chromatographic column manufactured by Bio-Rad Laboratories, Hercules, California, USA, to obtain four distinct peptide fragments. Analysis using amino acid sequencer revealed that these polypeptide fragments beared an amino acid sequence as represented by or

### Experiment 2-4

### Ultraviolet absorption spectrum

After measuring ultraviolet absorption spectrum in aqueous solution using spectrophotometer, the purified polypeptide showed an absorption maximum around a wave length of 280nm.

### Experiment 2-5

### Solubility in solvents

After testing in usual manner, the purified polypeptide was found to be soluble in water, physiological saline and phosphate buffer.

### Experiment 2-6

### Biological activities

When tested by the following methods, the purified polypeptide exhibits properties of combining immunoglobulin E antibodies from patients with pollen allergy, as well as of inducing proliferation of T cells which specifically react with the polypeptide.

### Experiment 2-6(a)

### Binding test on immunoglobulin E antibody

Onto each well in 96 well microplate was adsorbed 1 microliter/well of the purified polypeptide and added 1 microliter/well of a serum from pollen allergy patient or healthy volunteer, and the microplate was incubated at 37°C for 2 hours. The microplate was then washed with distilled water to remove non-adsorbed sera and each well was added with 1 microliter/well of goat anti-human immunoglobulin E antibody which had been labelled with horseradish peroxidase, followed by incubating at 37°C for additional 2 hours. After washing with distilled water for removal of non-adsorbed antibodies, each well on the microplate was added with small amounts of aqueous hydrogen peroxide solution containing 1,2-phenylenediamine for development and then determined for absorbance at 492nm.

As the result, the absorbance in the system where sera from healthy volunteer were used was about 0.1, while in the system where sera from patient with pollen allergy were used the absorbance reached to about 2.0, indicating that the purified polypeptide notably combined immunoglobulin E antibodies in blood from the patient. This would support that the purified polypeptide is one of the causative substances for pollen allergy, in other words, bearing a property of eliciting pollen allergy.

### Experiment 2-6(b)

### T cell proliferation-inducing test

Mononuclear cells were isolated from heparinized peripheral blood of a patient with pollen allergy using the Ficoll-Hypaque gradient contrifugation. The mononuclear cells were then suspended in RPMI 1640 medium (pH7.0) supplemented with 10% (v/v) AB serum to give a cell density of 1×10⁶ cells/ml, added with 20 micrograms/ml of the purified polypeptide obtained in Experiment 1 and cultured in 5% CO₂ incubator at 37°C for 7 days. Thereafter the cells were washed with RPMI 1640 medium (pH7.0) and resuspended in a fresh preparation of the the same medium to give a cell density of 1×10⁶ cells/ml. The medium was then added 5 units/ml of recombinant human IL 2 and cultured for additional 3 days under the same conditions as described above. The mononuclear cells pre-treated as above were subjected to the following T cell growth test.

On 96 well microplate was placed 2×10⁴ cells/well of the mononuclear cells in suspension in RPMI 1640 medium (pH7.0) supplemented with 10% (v/v) AB serum, 2 microCi of ³H-thymidine and 100 micrograms/ml of the purified polypeptide and the microplate was filled with physiological saline to 200 microliters/well. The cell in each well was cultured in 10% CO₂ incubator at 37°C for 3 days and determined with scintillation counter for intracellular incorporation of ³H-thymidine. At the same time an additional system as control using physiological saline which was free of the polypeptide was provided and treated similarly as above.

As the result, in the control about 300cpm of incorporation was observed, while the systems with the purified polypeptide showed a notable incorporation, i.e. about 6,500cpm per 100 micrograms/ml of the polypeptide. This would suggest that the purified polypeptide has an antigenicity.

### Experiment 2-7

### Stability

After incubating the purified polypeptide in aqueous solution (pH7.2) at 100°C for 10 minutes, no residual activity was detected. While after storing the purified polypeptide in aqueous solution (pH7.2) at 4°C for 1 month, no substantial decay in activity was detected.

Such polypeptide, which bears physicochemical properties as described above, has not been known and therefore is deemed to be a novel substance.

Now explaining several Experiments to determine both the nucleic acid sequence coding the polypeptide and its whole amino acid sequence, in sum, the polypeptide of this invention was sequenced through a series of operations as follows:
(1) An extract of pollen which had been collected from cedar male flower was subjected to purification including chromatography to isolate the polypeptide in a highly pure form.
(2) The polypeptide was then trypsinized and four distinct peptide fragments were isolated from the digest and determined for amino acid sequence.
(3) Several oligonucleotides which might code these partial amino acid sequences were chemically synthesized and then used as primers in RT-PCR where mRNAs which had been separately extracted from cedar pollen were used as template for gene amplification.
(4) Additional oligonucleotides as probes which had been chemically synthesized similarly as above with reference to the aforementioned partial amino acid sequences and then isotope-labelled were hybridized with the DNA fragments which had been obtained by the gene amplification, thus selecting a plurality of DNA fragments which partially coded the polypeptide.
(5) The DNA fragments were sequenced and an amino acid sequence which was decoded therefrom was compared with the aforementioned partial amino acid sequences, thus confirming that the presumed nucleic acid sequence did code the peptide.

### Experiment 3

### Determination of whole amino acid sequence of polypeptide and nucleic acid sequence coding the same

In this Experiment, both the nucleic acid sequence which codes the polypeptide and its whole amino acid sequence will be determined. The procedures used in this Experiment are known in the art and detailed, for example, in T. Maniatis et al., Molecular Cloning A Laboratory Manual, 1989, Cold Spring Harbor Laboratory Press, Cold Spring, New York, USA and M. Muramatsu, Labomanual Idenshi-kogaku (Laboratory Manual for Genetic Engineering), 1988, Maruzen Co., Ltd., Tokyo, Japan.

### Experiment 3-1

### Preparation of total RNA

Ten grams of cedar pollen which had been pretreated by lyophilization in liquefied nitrogen was added with both 40ml of a solution containing 10mM Tris-HCl buffer (pH7.4), 20mM sodium chloride, 6mM magnesium chloride and 1% (w/v) SDS and 40 ml of phenol/chloroform/isoamyl alcohol mixture solution and the mixture was treated with Polytron to crush the pollen. The resultant was centrifuged at 10,000×g and 4°C for 10 minutes and the aqueous layer was collected, added with the equivolume of phenol/chloroform/isoamyl alcohol mixture solution, extracted similarly as above and centrifuged again to obtain a newly formed aqueous layer which was then added with 2.5-fold volume of ethanol and allowed to stand at -20°C for 2 hours to effect sedimentation of the total RNA.

The total RNA was washed with 75% (v/v) ethanol, dried, dissolved in 29ml sterile distilled water, added with 300mM phosphate buffer (pH6.8) and then loaded to hydroxy-apatite column which had been equilibrated with 10mM phosphate buffer (pH6.8). The column was washed with 10mM phosphate buffer (pH6.8) and applied with 300mM phosphate buffer (pH6.8) to elute the total RNA which was then added with 2.5-fold volume of 75% (v/v) ethanol and allowed to stand at -20°C for 2 hours to effect sedimentation of the total RNA. The total RNA was then washed with 75% (v/v) ethanol, dried and dissolved in 0.5ml sterile distilled water for use in selection of the following clones SC09, SC31, SC216, SC50 and SC362. The recovery yield for total RNA was about 500 micrograms.

### Experiment 3-2

### Preparation of first strand cDNA

One microgram of the total RNA obtained in Experiment 3-1 was added with 4 microliters of 25mM magnesium chloride, 2 microliters of 10×PCR buffer comprising 500mM potassium chloride in 10mM Tris-HCl buffer (pH8.3), 8 microliters of dNTP mix, 1 microliter of RNase inhibitor (1 unit/microliter), 1 microliter of reverse transcriptase (2.5 units/microliter) and 1 microliter of 2.5 micromole random hexamer and filled up with sterile distilled water to 20 microliters. The mixture was incubated in usual manner in 0.5ml-reaction vessel at 25°C for 10 minutes, 42°C for 30 minutes, 99°C for 5 minutes and 5°C for 5 minutes in the given order to effect reverse transcriptase reaction, thus obtaining a solution which contained a first strand cDNA.

### Experiment 3-3

### Selection of clone SC09 and determination of partial nucleic acid sequence

Twenty microliters of the first strand cDNA in solution obtained in Experiment 3-2 was added with 4 microliters of 25mM magnesium chloride, 8 microliters of 10×PCR buffer comprising 500mM potassium chloride in 100mM Tris-HCl buffer (pH8.3), 0.5 microliters of Taq DNA polymerase (2.5 units/microliter) and 1 picomole each of Primers 1 and 2 as sense and antisense primers respectively which will be described below and filled up in sterile distilled water to 100 microliters. The mixture was then allowed to react 40 times in usual manner at a cycle of 94°C for 1 minute, 45°C for 2 minutes and 72°C for 3 minutes, thus amplifying DNA fragments which partially coded the polypeptide using the cDNA as template. Primers 1 and 2 were oligonucleotides with nucleic acid sequences as represented by Tables 1 and 2 respectively which were both chemically synthesized in accordance with either His-Asp-Ala-Ile-Asn-Ile or Ala-Trp-Gln-Ala-Ala in the partial amino acid sequences (a) and (b) in Experiment 2-3. The PCR reagent mainly used in the above was "GeneAmp RNA PCR Kit" commercialized by Takara Shuzo Co., Ltd., Shiga, Japan.

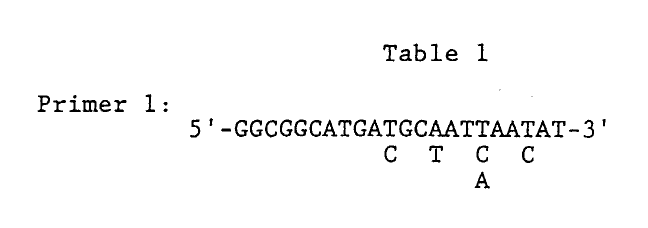

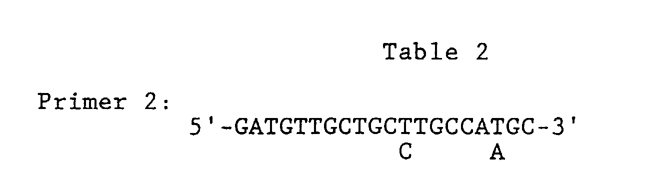

A portion of the resultant PCR product was electrophoresed in usual manner on 2% agarose gel for fractionation, blotted on a nylon membrane and fixed thereon with 0.4N sodium hydroxide. The nylon membrane was washed with 2×SSC, dried in air stream, soaked in a mixture solution for prehybridization containing 5×SSPE, 5×Denhardt solution, 0.5% (w/v) SDS and 100 micrograms/ml of denatured salmon sperm DNA and incubated at 65°C for 3 hours. Separately an oligonucleotide as Probe 1 with a nucleic acid sequence as shown in Table 3 which might code Phe-Asn-Val-Glu-Lys in the partial amino acid sequence (a) in Experiment 2-3 was chemically synthesized in usual manner and labelled with [gamma-³²P] ATP using T4 nucleotide kinase. The nylon membrane was soaked in a mixture solution containing 5×SSPE, 5×Denhardt solution and 0.5% (w/v) SDS along with 1 picomole of Primer 1, incubated at 45°C for 24 hours for hybridization, washed with 6×SSC and autoradiographed to confirm the presence of the objective DNA fragment in the PCR product.

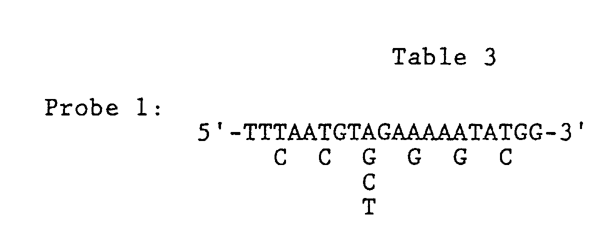

The remaining PCR product was added with 50ng of "pT7 Blue T", a plasmid vector commercialized by Takara Shuzo Co., Ltd., Shiga, Japan and an appropriate amount of T4 DNA ligase, further added with 100mM ATP to give a final concentration of 1mM and incubated at 16°C for 24 hours to insert the DNA fragment in the plasmid vector, followed by introducing the resultant recombinant DNA in "NoVa Blue" strain of Escherichia coli commercialized by Pharmacia Fine Chemicals AB, Uppsara, Sweden. The resultant transformants were inoculated on a plate medium comprising 10g/l of bacto trypton, 11g/l of sodium chloride, 15g/l of bacto agar, 100mg/l of ampicillin, 40mg/l of X-Gal and 23.8mg/l of isopropyl-beta-D-thio galctopyranoside (abbreviated as "IPTG" hereinafter) and incubated at 37°C for 24 hours to allow the transformants to form colonies on the plate medium. Thereafter a fresh nylon membrane was placed onto the plate medium, allowed to stand for 30 seconds, put off and soaked for 7 minutes in a mixture solution containing 0.5M sodium hydroxide and 1.5M sodium chloride. The nylon membrane was then put out from the mixture solution, further soaked for 3 minutes in 0.5M Tris-HCl buffer (pH7.2) containing 1.5M sodium chloride, washed with 2×SSC, soaked again for 20 minutes in 0.4M sodium hydroxide, washed with 5×SSC, dried in air stream, soaked in a mixture solution for prehybridization containing 5×SSPE, 5×Denhardt solution, 0.5% (w/v) SDS, 100 micrograms/ml of denatured salmon sperm DNA and incubated at 65°C for 3 hours. The nylon membrane was then hybridized similarly as above with Probe 1 and autoradiographed and with reference to the resultant autoradiograph a clone SC09 which contained the objective DNA fragment was selected from the plate medium.

The clone SC09 was inoculated in L-broth medium (pH7.2) containing 100 micrograms/ml of ampicillin and cultured at 37°C for 18 hours and the cells were collected from the resultant culture, crushed and extracted by usual alkali method to obtain a recombinant DNA. The DNA fragment in the recombinant DNA was sequenced by the dideoxy method using "Sequenase Version 2.0", a DNA sequencing kit commercialized by Toyobo Co., Ltd., Tokyo, Japan, confirming that the DNA fragment beared the 173rd through 240th nucleic acids in the sequence in the Sequence Listing with SEQ ID No.2.

### Experiment 3-4

### Selection of clone SC31 and determination of partial nucleic acid sequence

In this Experiment, another clone SC31 with a DNA fragment different from that in the clone SC09 was selected and its nucleic acid sequence was determined. Respective steps in this Experiment were the same as used in Experiment 3-3 as specified otherwise.

In usual manner, both Primer 3 with a nucleic acid sequence as shown in Table 4 which had been arbitrarily chosen from the partial nucleic acid sequence in the clone SC09 and Primer 4 with a nucleic acid sequence as shown in Table 4 which might code Asn-Ile-Val-Ile-Glu in the partial amino acid sequence (c) in Experiment 2-3 were chemically synthesized. A portion of the first strand cDNA in solution obtained in Experiment 3-2 was subjected to gene amplification similarly as in Experiment 3-3 in the presence of Primers 3 and 4 and from the resultant DNA fragments was collected another DNA fragment which partially coded the polypeptide using Probe 2 with an amino acid sequence shown in Table 6 which had been arbitrarily chosen from the partial nucleic acid sequence in the clone SC09. Thereafter, similarly as in Experiment 3-3, a fresh preparation of the plasmid vector "pT7 Blue T" which had been inserted with the DNA fragment was introduced in "NoVa Blue" strain of Escherichia coli and from colonies which were obtained by culturing the strain was selected another clone SC31 using Probe 2. The clone SC31 beared a nucleic acid sequence which was correspondent to the 210th through 795th nucleic acids in the sequence in the Sequence Listing with SEQ ID No.2.

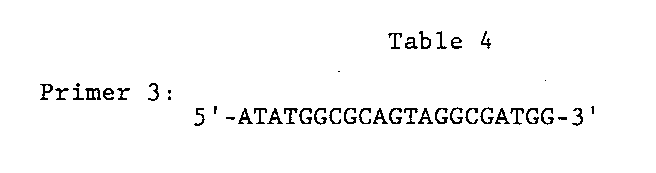

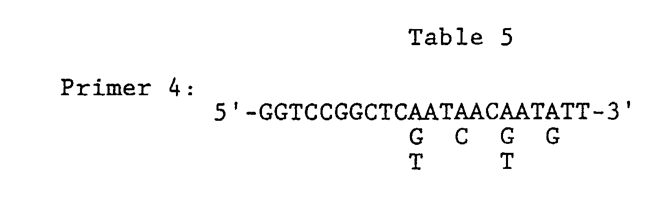

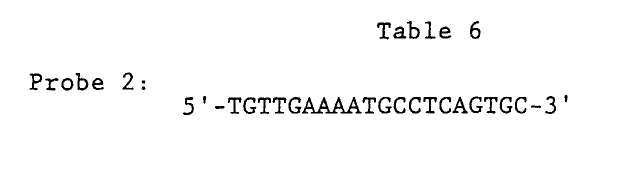

### Experiment 3-5

### Selection of clone SC216 and determination of partial nucleic acid sequence

In usual manner, both Primer 5 with a nucleic acid sequence as shown in Table 7 which had been arbitrarily chosen from the partial nucleic acid sequence in the clone SC31 selected in Experiment 3-4 and Primer 6 with a nucleic acid sequence as shown in Table 8 which might code Asn-Asp-Asn-Ala-Asn-Gly in the partial amino acid sequence (d) in Experiment 2-3 were chemically synthesized. A portion of the first strand cDNA in solution obtained in Experiment 3-2 was subjected to gene amplification similarly as in Experiment 3-3 in the presence of Primers 5 and 6 and from the resultant DNA fragments was selected still another DNA fragment which partially coded the polypeptide using Probe 3 with a nucleic acid sequence as shown in Table 9 which had been arbitrarily chosen from the partial nucleic acid sequence in the clone SC31. Thereafter, similarly as in Experiment 3-3, a fresh preparation of the plasmid vector "pT7 Blue T" which had been inserted with the DNA fragment was introduced in "NoVa Blue" strain of Escherichia coli and from colonies which were obtained by culturing the strain was selected still another clone SC216 using Probe 3. The clone SC216 beared a nucleic acid sequence which was correspondent to the 763rd through 1,233rd nucleic acids in the sequence in the Sequence Listing with SEQ ID No.2.

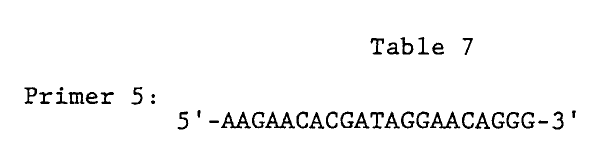

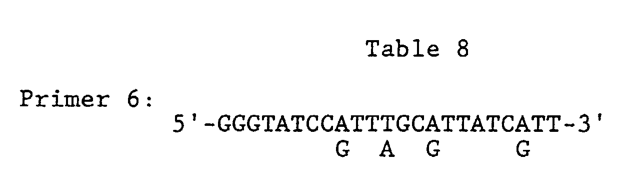

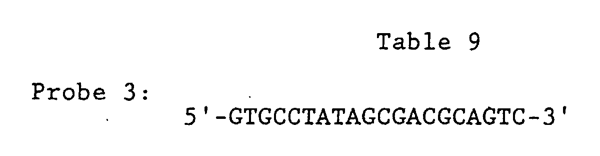

### Experiment 3-6

### Selection of clone SC50 and determination of partial nucleic acid sequence

A portion of the total RNA obtained in Experiment 3-1 was subjected to reverse transcriptase reaction in accordance with the method in Experiment 3-2 in the presence of Primer 7 with a nucleic acid sequence as shown in Table 10 which was arbitrarily chosen from the partial nucleic acid sequence in the clone SC09. The resultant first strand cDNA in solution was added with 4 microliters of 5xtailing buffer, 4 microliters of dATP and 10 units of deoxyribonucleic acid terminal transferase, filled up with sterile distilled water to 20 microliters and incubated at 37°C, 70°C and 4°C in the given order for 5 minutes each. After removal of the residual primer from the reaction product with spin filter and subsequent concentration, the resultant poly(dA)-attached first strand cDNA was added with 10 picomoles of Primer 8 with a nucleic acid sequence as shown in Table 11 which was reported by M.A. Frohman et al. in Proceedings of the National Academy of Sciences USA, Vol.85, pp.8,998-9,002 (1988), 10 microliters of 10×PCR buffer, 1 microliter of dNTP mix and 0.5 microliters of Taq DNA polymerase (2.5 units/microliter), filled up with sterile distilled water to 100 microliters and incubated at 72°C for 30 minutes, 99°C for 5 minutes and 4°C for 5 minutes in the given order to synthesize a second strand cDNA. After removing the residual primer, the reaction product was concentrated, added with 10 picomoles of Primer 9 with a nucleic acid sequence as shown in Table 12 which was reported by Frohman et al. in the same article as above, 10 picomoles of Primer 10 with a nucleic acid sequence as shown in Table 13 which had been arbitrarily chosen from the partial nucleic acid sequence in the clone SC09, 10 microliters of 10×PCR buffer, 1 microliter of 1mM dNTP mix and 0.5 microliters of Taq DNA polymerase (2.5 units/microliter), filled up with sterile distilled water to 100 microliters and subjected to gene amplification similarly as in Experiment 3-3, and from the resultant DNA fragments was selected still another DNA fragment which partially coded the polypeptide using Probe 4 with a nucleic acid sequence as shown in Table 14 which had been arbitrarily chosen from the partial nucleic acid sequence in the clone SC09. Thereafter, similarly as in Experiment 3-3, a fresh preparation of the plasmid vector "pT7 Blue T" which had been inserted with the DNA fragment was introduced in "NoVa Blue" strain of Escherichia coli and from colonies which were obtained by culturing the strain was selected still another clone SC50 using Probe 4. The clone SC50 beared a nucleic acid sequence which was correspondent to the first through 198th nucleic acids in the sequence in the Sequence Listing with SEQ ID No.2.

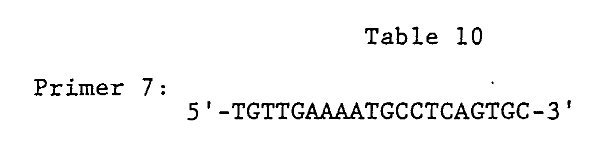

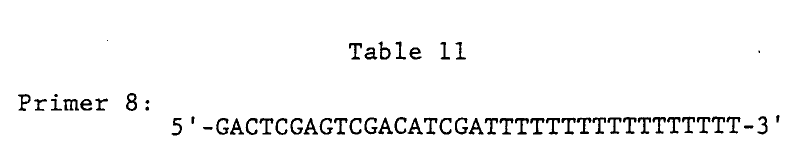

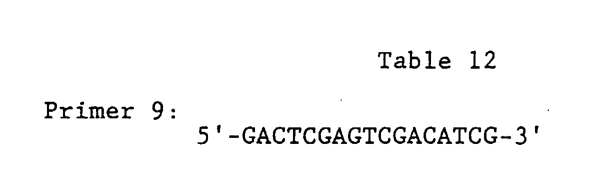

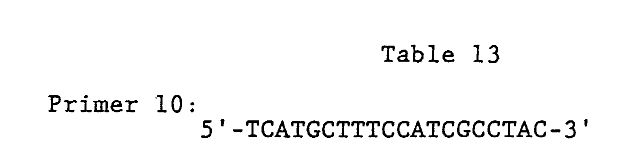

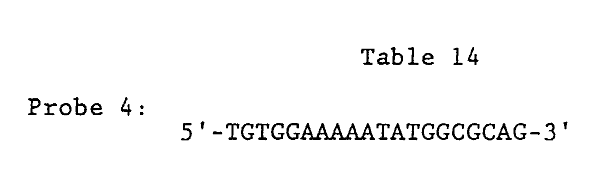

### Experiment 3-7

### Selection of clone SC362 and determination of partial nucleic acid sequence

Twenty microliters of the total RNA obtained in Experiment 3-1 was subjected to reverse transcriptase reaction similarly as in Experiment 3-2 except that the random hexamer was replaced with 0.5 micrograms of Primer 8 thus obtaining a solution which contained a first strand cDNA. The solution was subjected to gene amplification in accordance with the method in Experiment 3-3 in the presence of Primer 9 as shown in Table 12 and Primer 11 with a nucleic acid sequence as shown in Table 15 which had been arbitrarily chosen from the partial nucleic acid sequences in the clone SC216, and from the resultant DNA fragments was selected still another DNA fragment which partially coded the polypeptide using Probe 5 with a nucleic acid sequence as shown in Table 16 which had been arbitrarily chosen from the partial nucleic acid sequence in the clone SC216. Thereafter, similarly as in Experiment 3-3, a fresh preparation of the plasmid vector "pT7 Blue T" which had been inserted with the DNA fragment was introduced in "NoVa Blue" strain of Escherichia coli and from colonies which were obtained by culturing the strain was selected still another clone SC362 using Probe 5. The clone SC362 beared a nucleic acid sequence which was correspondent to the 1,216th through 1,545th nucleic acids in the sequence in the Sequence Listing with SEQ ID No.2.

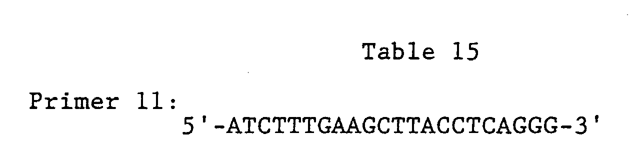

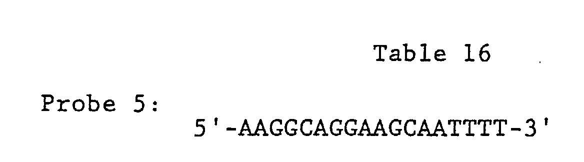

### Experiment 3-8

### Whole nucleic acid sequence coding polypeptide and its whole amino acid sequence

After analyzing all the results in Experiments 3-3 through 3-7, it was found that the gene coding the polypeptide beared the nucleic acid sequence starting at 5'-terminal in the Sequence Listing with SEQ ID No.3. Decoding of this nucleic acid sequence revealed that the polypeptide beared the amino acid sequence starting at N-terminal in the Sequence Listing with SEQ ID No.3 which was composed of 514 amino acid residues at maximum and of course contained all of the four types of partial amino acid sequences in Experiment 2-3.

As described above, the polypeptide of this invention is discovered first in the world by us as a crop of long-term research, which bears distinct physicochemical properties and amino acid sequence which are not seen in conventional cedar pollen allergens. While advances in recombinant DNA technology in the art have however made it possible to replace one or more constitutive amino acids with other amino acids without substantially changing biological properties of polypeptides. In view of such technical level, the polypeptide as referred to in this invention shall include, in addition to those which bear the same amino acid sequence as shown in the Sequence Listing with SEQ ID No.1, all the mutants where one or more amino acids in the amino acid sequence are replaced with other amino acids while retaining the specified N-terminal amino acid sequence. Thus the polypeptide of this invention includes all the polypeptides which are derived from natural sources and artificially synthesized, as long as they do bear the amino acid sequence in the Sequence Listing with SEQ ID No.1 or an amino acid sequence homologous therewith. Examples of such a natural source include cedar plants of Cryptomeria japonica and as described above from their pollens and male flowers can be obtained the polypeptide of this invention and genes which code the same. To artificially synthesize the polypeptide of this invention, for example, chemical synthesis is carried out with reference to the nucleic acid sequence in the Sequence Listing with SEQ ID No.2 and alternatively DNAs which bear the same nucleic acid sequence are inserted in appropriate expression vectors and introduced in recombinant DNA forms into appropriate hosts to obtain transformants which are then subjected to cultivation.

The DNA of this invention shall include, in addition to those which bear the same nucleic acid sequence as shown in the Sequence Listing with SEQ ID No.2, other DNAs with a nucleic acid sequence which differs in one or more nucleic acids, as long as such a DNA does code the amino acid sequence as shown in the Sequence Listing with SEQ ID No.1.

Further in the art degeneracy in genetic codes makes it possible to replace one or more nucleic acids in DNAs without changing the amino acid sequences they code. Because of this, the DNA of this invention shall include, in addition to those which bear the same nucleic acid sequence as shown in the Sequence Listing with SEQ ID No.2, other DNAs which bear a nucleic acid sequence where based on degeneracy of genetic codes one or more nucleic acids are replaced with other nucleic acids, as long as they do code the polypeptide with the amino acid sequence as shown in the Sequence Listing with SEQ ID No.1 or its homologous mutants.

Still further in accordance with the present recombinant DNA technology, generally, when a nucleic acid sequence starting at 5'-terminal is one determined, the its complementary sequence is uniquely determined. Thus the DNA of this invention shall include all the nucleic acid sequences which are complementary with either of the above. Of course one or more nucleic acids in the nucleic acid sequences which code the polypeptide or its homologous mutants can be replaced with other nucleic acids so that the DNA of this invention actually expresses the production of the polypeptide in hosts.

As described above, this invention is to provide a process to produce the polypeptide wherein recombinant DNA technology is applied. The process according to this invention comprises cultivating in a culture medium a transformant which contains a DNA coding the polypeptide, and collecting the produced polypeptide from the resultant culture. The transformants feasible in this invention usually contain a replicable recombinant DNA wherein a DNA which codes the polypeptide has been inserted. Such recombinant DNA, which usually comprises a cDNA coding the polypeptide and a replicable expression vector, can be easily prepared by usual recombinant DNA technology as long as such DNA is available. Examples of such expression vector include pKK223-3, pRL-lambda, pBTrp2 DNA, pUB110, YEpl3, Ti plasmid, Ri plasmid and pBI121: Among these, pKK223-3, pRL-lambda, pBTrp2 DNA, pUB110 and YEpl3 are suitable to allow the DNA of this invention to express in microorganisms such as Escherichia coli, Bacillus subtilis and yeast, while to express in plant cells Ti and Ri plasmids are feasible.

To insert the DNA of this invention in expression vectors, a variety of methods which are common in the art are feasible. For example, a DNA which contains the DNA of this invention is cleaved with appropriate restriction enzymes, while an appropriate replicable expression vector is also cleaved with restriction enzymes of the same types. Then the resultant DNA and vector fragments are linked with DNA ligase, thus obtaining a replicable recombinant DNA. The transformant of this invention which is obtainable by introducing such recombinant DNA in microorganisms such as Escherichia coli, Bacillus subtilis, ray fungi and yeast or in cells of higher animals and plants by the competent cell method, protoplast method or electroporation method intracellularly or extracellularly produces the polypeptide when cultivated in culture media.

The produced polypeptide can be purified by methods which are common in the art. For example, microorganisms or cells are collected from a culture of the transformant of this invention and, if necessary, crushed, after which the resultant crude polypeptide is purified by centrifugation, salting out, dialysis, column chromatography, high-performance liquid chromatography, affinity chromatography and/or electrophoresis. In particular, monoclonal antibodies specific to Cry j II as reported in T. Kawashima et al., International Archives of Allergy and Immunology, Vol.98, pp.110-117 (1992) are very useful in the purification of the polypeptide and the use of affinity chromatographies using water-insoluble carriers binding such a monoclonal antibody minimizes labor and time needed to obtain the polypeptide in highly pure form. Partially-purified or purified polypeptides obtained in this way will find extensive uses as desensitizer directed to treatment, prevention and/or diagnosis of pollen allergy, as well as in scientific researches to reveal the incident mechanisms of allergic diseases in general including pollen allergy.

Several embodiments will be given hereinafter to illustrate the process to produce polypeptide according to this invention.

### Example A-1

### Production of polypeptide

This Example is to illustrate the production of the polypeptide wherein a DNA which codes the polypeptide is inserted in Escherichia coli and the resultant transformant is subjected to cultivation. Such procedures can be however modified in various ways in the art, therefore this Example is just an embodiment and not intended to restrict the scope of this inevention thereto.

### Example A-1(a)

### Preparation of transformant

One microgram of the total RNA obtained in Experiment 3-1 was subjected to gene amplification similarly as in Experiments 3-2 and 3-3 except that the sense and antisense primers were replaced with Primer 12 with a nucleic acid sequence as shown in Table 17 and Primer 13 with a nucleic acid sequence as shown in Table 18 which had been arbitrarily chosen from the partial nucleic acid sequences in the clones SC50 and SC216 respectively. Thereafter the PCR product containing DNA fragments were cleaved in usual manner with restriction enzymes Eco RI and Nco I to obtain DNA fragments with initiator codon ATG wherein Eco RI and Nco I restriction sites were linked.

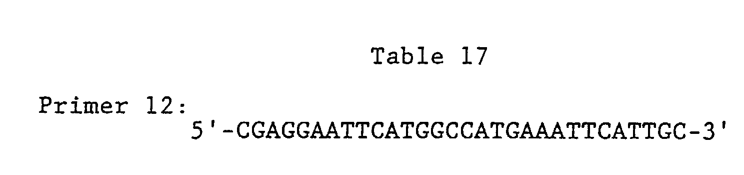

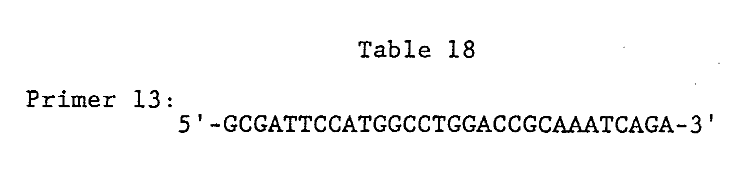

Separately one microgram of the total RNA obtained in Experiment 3-1 was subjected to genetic amplification similarly as in Experiments 3-2 and 3-3 except that the sense and antisense primers were replaced with Primers 14 and 15 with nucleic acid sequences as shown in Tables 19 and 20 which had been arbitrarily chosen from the partial nucleic acid sequences in the clones SC216 and SC362 respectively. Thereafter the PCR product containing DNA fragments were cleaved in usual manner with restriction enzymes Pst I and Nco I to obtain DNA fragments with terminator codon TGA wherein Pst I and Nco I restriction sites were linked.

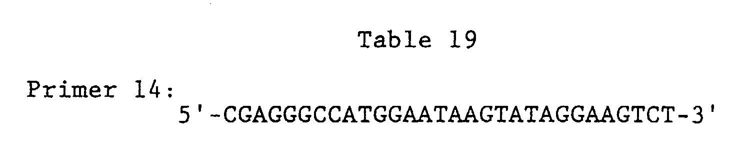

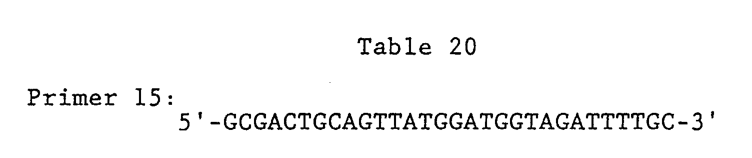

Ten gram each of these two types of DNA fragments obtained in this way were added in an appropriate amount of sterile distilled water with 10ng of "pKK223-3", a plasmid vector commercialized by Pharmacia Fine Chemicals AB, Uppsara, Sweden, which had been cleaved with restriction enzymes Eco RI and Pst I, along with an appropriate amount of T4 DNA ligase, further added with 100mM ATP to give a final concentration of 1mM and incubated at 16°C for 18 hours. The obtained recombinant DNA was introduced by the competent cell method in JM109 strain of Escherichia coli (ATCC 53323) for transformation. The resultant transformant was inoculated in usual manner in L-broth medium (pH7.2) containing 100 micrograms/ml of ampicillin and cultured at 37°C for 18 hours, after which the cells were centrifugally collected from the resultant culture and subjected to usual alkali method to extract a recombinant DNA. This recombinant DNA was designated as "pKCJ2-5" and analyzed for structure, leading to the finding that as seen in FIG.1 in pKCJ2-5 a nucleic acid sequence KCJ cDNA which was equivalent with that in the Sequence Listing with SEQ ID No.2 was linked downstream at Tac promoter Ptac.

### Example A-1(b)

### Production of polypeptide

The transformant obtained in Example A-1(a) was inoculated in L-broth medium (pH7.2) containing 100 micrograms/ml of ampicillin and cultured at 37°C for 18 hours, after which 0.1ml of the culture was transferred to a fresh preparation of the same medium and further cultivated at 37°C. When the absorbance of the medium at a wave length of 600nm reached 0.3, the medium was added with IPTG to give a final concentration of 1mM and cultivated for additional 4 hours. Thereafter the cells were centrifugally collected from the culture, suspended in chilled distilled water, ultrasonically crushed and centrifuged, followed by collecting the supernatant.

The supernatant was concentrated and loaded to mAbN26 Sepharose column binding a monoclonal anti-Cry j II antibody which had been equilibrated with 0.14M sodium chloride in 10mM phosphate buffer (pH7.0), followed by applying to the column 0.1M glycine-HCl buffer (pH2.3) to elute peptide components. Fractions which contained the polypeptide were collected, neutralized by the addition of 1M phosphate buffer (pH7.0) and dialyzed against 10mM phosphate buffer (pH7.0) for 10 hours. Thereafter the solution inside dialyzing means was collected, concentrated and lyophilized in usual manner to obtain a solid of purified polypeptide. The yield was about 3mg per 1 liter of the culture medium.

### Example A-1(c)

### Physicochemical properties of polypeptide

After testing by the methods in Experiments 2-1 and 2-2, the polypeptide obtained by the method in Example A-1(b) was found to bear the following physicochemical properties:
(1) Molecular weight
   After testing on SDS-polyacrylamide gel electrophoresis similarly as in Experiment 2-1, the purified polypeptide gave a major band correspondent to a molecular weight of 55,000±5,000 daltons.
(2) Isoelectric point
   After testing on isoelectric point electrophoresis similarly as in Experiment 2-2, the purified polypeptide gave an isoelectric point at 9.0±0.5.
(3) N-Terminal amino acid sequence
   After analyzing in usual manner using "Model 470A", an amino acid sequencer commercialized by Perkin-Elmer Corp., Foster city, California, USA, the purified polypeptide was found to bear at N-terminal an amino acid sequence as represented by Ala-Met-Lys-Phe-Ile-Ala-Pro-Met-Ala-Phe-Val-Ala-Met-Gln-Leu-Ile-Ile-Met-Ala-.
(4) Ultraviolet absorption spectrum
   After determining using spectrophotometer for ultraviolet absorption spectrum in aqueous solution, the purified polypeptide gave an absorption maximum around a wave length of 280nm.
(5) Solubility in solvents
   After testing in usual manner, the purified polypeptide was found to be soluble in water, physiological saline and phosphate buffer.
(6) Biological activity
   After testing by the method in Experiment 2-6(a), the absorbance in a system using sera from healthy volunteer was about 0.1, while in another system using sera from patient with pollen allergy the absorbance reached about 2.0, indicating that the polypeptide combined immunoglobulin E antibodies in blood of the patient. This would support that the polypeptide is one of the causative substances of pollen allergy, in other words, having a property of eliciting pollen allergy.
   While after testing by the method in Experiment 2-6(b), about 200cpm incorporation was marked in control system where physiological saline without the polypeptide was administered, while in a system with the purified polypeptide was administered marked a notable incorporation, i.e. about 8,500cpm per 50 micrograms/ml of the polypeptide. This would suggest that the polypeptide has an antigenicity.

The use of the polypeptide according to this invention will be concretely explained hereinafter in conjunction with several Examples and Experiments.

The polypeptide of this invention would have extensive uses as desensitizer for treatment, prevention and diagnosis of pollen allergy because it is one of the causative substances for pollen allergy. The desensitizer of this invention comprises as effective ingredient either the polypeptide or a conjugate with specific saccharides which will be explained below. In desensitizers for diagnosis, generally, partially-purified or purified polypeptides are incorporated intact, while in case of treating or preventing pollen allergy, such a polypeptide is covalently bound to specific saccharides, prior to incorporation.

The saccharide as referred to in this invention means those which enable covalent binding with the polypeptide and then enhance and/or reduce its desensitizing activity and possible side effects respectively. Such a saccharide is feasible, for example, with simple or complex polysaccharides such as starch, amylose, dextran, plysucrose, pullulan, elsinan, curdlan, gum arabic, tragacanth gum, guar gum, xanthan gum, carageenan, cellulose, glucomannan, chitosan, lipopolysaccharides and their derivatives and partial hydrolysates which usually bear averaged molecular weights in the range of about 500-10,000,000 daltons, desirably, about 10,000-1,000,000 daltons. In particular, when administered in mammals including human, conjugates with pullulan, elsinan or their partial hydrolysate are notable in properties of enhancing the production of immunoglobulin G and M antibodies in high level which are effective in desensitization, as well as of producing less immunoglobulin E antibody which is one of the major causes of side effects including anaphylactic shock. These are very favorable in safe and effectively practicing desensitizing therapies where repeated administration is indispensable.

Further, lipopolysaccharides and their partial hydrolysates from certain microorganisms, for example, of Escherichia coli, Salmonella and Serratia have properties of enhancing affinities of the polypeptide to mucous membranes in mammals and then significantly improving its absorption efficiency. This renders conjugates with these saccharides very useful in desensitizers which are mainly administered through percutaneous or permucosal routs.

The conjugates as described above are usually obtainable either by allowing the polypeptide with activated saccharides or by crosslinking the polypeptide and saccharides with reagents which bear two or more functional groups in molecule. Particular reactions methods are, for example, diazo method, peptide method, alkylation method, crosslinking method, periodate oxidation method and disulphide method which are known in the art: Typical methods are detailed, for example, in Japan Patent Kokai No.93,730/91. The ratio of the polypeptide and saccharide at the starting of reaction is set in the range of about 1:0.001-1:1,000, desirably, about 1:0.01-1:100 by weight. Dependently on particular reaction methods, when the ratio is below the range, linkages between peptide molecules become notable, while linkages between saccharide molecules become notable when the ratio exceeds the range. In either case efficiencies in reaction and postreaction purification are decreased, therefore the above range was judged most preferable. Reaction time and temperature and pH during reaction are chosen in such manner that inactivation and decomposition of the polypeptide and undesirable side reactions are minimized: Temperature and pH are set to about 0-100°C and about 0.1-12 respectively so that reaction is completed within 0.1-50 hours.

Conjugates formed by the reactions can be purified by methods which are common in the art, for example, dialysis, salting out, filtration, concentration, centrifugation, gel filtration chromatography, ion exchange chromatography, affinity chromatography, gel electrophoresis and isoelectric point electrophoresis which may be arbitrarily combined if necessary. Purified conjugates are concentrated and lyophilized into liquid or solid form dependently on needs in final uses.

The desensitizer of this invention includes, in addition to those in form where the polypeptide and/or conjugate is solely used, those in composition, for example, with physiologically-acceptable carrier, vehicle, diluent, adjuvant and/or stabilizer, as well as with one or more other medicinal agents including anti-inflammatory and anti-histamine agents such as steloid hormones and disodium cromoglycate. The desensitizer of this invention also includes medicinal agents in unit forms which are devised to meet to particular medications: This wording means those which contain the polypeptide and/or conjugate of this invention in an amount correspondent to either one day dose or its multiple (up to 4-fold) or divisor (up to 1/40-fold) and which are prepared into one body so that it can be physically separated when in medication. Examples of such a form include powder, parvule, granule, pilula, tablet, capsule, lozenge, syrup, emulsion, ointment, emplastrum, cataplasm, bougie, collyrium, collunarium, nebula and injection.

Now explaining the way of using the desensitizer of this invention, generally, the desensitizer of this invention is used similarly as conventional desensitizers are. More particularly in diagnosis of pollen allergy, certain test methods which are known in the art as scratch or intradermal test are used: More particularly, on the surface of subjects' skin one or more scratches small enough not to bleed are made and an appropriate amount of a diagnostic desensitizer according to this invention is dropped thereto. Alternatively an appropriate amount of such desensitizer is intradermally injected in subjects. In either case, after a lapse of 15-30 minutes the occurrence and size of urticaria are checked and if urticaria are larger than prescribed size, then the subjects are diagnosed as positive for pollen allergy.

In treatment using the desensitizer of this invention, generally, appropriate doses and administration routs are determined with reference to results in the aforementioned diagnosis. Subjects who have been diagnosed positive are orally or parenterally administered with desensitizers which contain conjugates of the polypeptide and specific saccharides according to this invention. Dependently on symptoms and administration routs, in particular, while carefully monitoring subjects' symptom and postadministration progress, generally, they are repeatedly administered through intradermal, subcutaneous, intramuscular, intraperitoneally or permucosal rout in an increasing dose within about 0.0001-100,000ng/adult, desirably, about 0.001-10,000ng/adult at a frequency of one time per week or month over a period of about one month to one year. Diagnosis of pollen allergy can be carried out with approximately the same dose and administration rout as above: While carefully monitoring subjects' physical conditions and postadministration progress, generally, they are repeatedly administered through intradermal, subcutaneous, intramuscular or permucosal rout in an increasing dose within about 0.0001-100,000ng/adult, desirably, about 0.001-10,000ng/adult at a frequency of one time per week or month over a period of about 1-6 months. When repeatedly administered at regular intervals from early autumn through early spring in the next year, possible allergic symptoms in the next incident season will be minimized or even eliminated.

The desensitizer of this invention will be concretely explained hereinafter in conjunction with several embodiments.

### Example B-1

### Dried injection

Two grams of a purified pullulan, averaged molecular weight of about 200,000 daltons, was dissolved in 100ml of distilled water and added with 1.7% (w/v) cyanulic chloride in acetone. The mixture was put in ice-chilled bath and allowed to stand at 4°C or lower for 2 hours while retaining the pH in the mixture around 7. The resultant solution which contained an activated pullulan was added with 200mg of a purified polypeptide obtained by the method in Example A-1(b) and allowed to react at pH7.0 and 37°C for 5 hours while stirring. Thereafter the reaction mixture was added with 1% (w/v) glycine and incubated at 37°C for one hour while stirring so that uncombined activated groups were blocked, dialyzed against 0.1M phosphate buffer (pH5.0) for 5 hours and applied to CM-Sephadex column chromatography which had been equilibrated with 0.01M phosphate buffer (pH5.0), followed by collecting from non-adsorbed fractions a conjugate of the polypeptide and pullulan. Thereafter in usual manner the conjugate was dissolved in physiological saline containing 1% (w/v) human serum albumin as stabilizer to give a final polypeptide concentration of about 100ng/ml, sterilely filtered, distributed in 2ml aliquots in sterile virals, lyophilized and sealed.

The product is added with 1ml distilled water for injection and then dissolved in vial to homogeneity, prior to use. The product, which contains a conjugate of the polypeptide and pullulan, is superior in stability and very useful as dried injection to treat and prevent pollen allergy.

### Example B-2

### Injection

One gram of CM-cellulose, averaged molecular weight of about 20,000 daltons, was dissolved in 200ml distilled water, added with 2g of 1-ethyl-3-(3-dimethyl aminopropyl) carbodiimide and allowed to react at ambient temperature for 2 hours while stirring and retaining the pH in the mixture around 4. The reaction mixture was then dialyzed against distilled water for 24 hours and the solution inside the dialyzing means was collected, added with 30mg of a purified polypeptide obtained by the method in Example A-1(b) and allowed to stand at ambient temperature and pH4.5 for 15 hours for reaction. Thereafter the conjugate in the reaction mixture was purified similarly as in Example B-1, concentrated, dissolved in 50% (v/v) aqueous glycerine solution, sterilely filtered, distributed in 2ml aliquots in sterile vials and sealed.

The product is added in vial with 100-100,000-fold volume of 50% (v/v) aqueous glycerine solution with reference to results in scratch and/or intradermal test and diluted to homogeneity, prior to use. The product, which contains a conjugate of the polypeptide and CM-cellulose, is useful as desensitizing injection to treat and prevent pollen allergy.

### Example B-3

### Liquid agent

One hundred milligrams of a purified lipopolysaccharide from Salmonella was dissolved in 25ml aqueous sodium acetate solution of 50% saturation, adjusted to pH9.0 with 0.5N sodium hydroxide and added dropwise with lml anhydrous dioxane containing 20 microliters of bromoacetyl bromide while retaining the pH in the reaction mixture around 8.5. The reaction mixture was then adjusted to about pH4.5 with 6N hydrochloric acid and dialyzed against 4°C distilled water for 48 hours to obtain a solution of activated lipopolysaccharide. The solution was added with 40mg of the purified polypeptide obtained by the method in Example A-1(b) and allowed to stand at ambient temperature for 48 hours for reaction while retaining the pH in the solution at about 4.5. The reaction product was purified, concentrated and lyophilized similarly as in Example B-1 to obtain a solid conjugate of the polypeptide and lipopolysaccharide. Thereafter the solid was dissolved in distilled water containing 1% (w/v) purified gelatin as stabilizer and sterilely filtered in usual manner to obtain a liquid agent.

The product, which contains as effective ingredient a conjugate of the polypeptide and lipopolysaccharide, is useful as liquid agent for collyrium, collunarium and intranasal nebula to treat and prevent pollen allergy.

### Example B-4

### Lozenge

Two grams of a purified elsinan, averaged molecular weight of 200,000 daltons, was dissolved in 400ml distilled water to homogeneity, adjusted to pH10.7 with 1N aqueous sodium hydroxide solution and gradually added with 3g cyanogen bromide over one hour for reaction while retaining the pH in the solution around 10.0. The reaction mixture was adjusted to pH5.0 with 1N hydrochloric acid and dialyzed against chilled water for 10 hours while retaining this pH level to obtain an aqueous solution of activated elsinan. The solution was then added with 20mg of a purified polypeptide obtained by the method in Example A-1(b) and allowed to stand at ambient temperature for 24 hours for reaction. After completion of the reaction, the reaction mixture was added with 3-fold volume of acetone and subjected to centrifugation to obtain a sediment which was then dissolved in 0.01M acetate buffer (pH5.0), centrifugally separated from insoluble substances and loaded to CM-Sephadex column chromatography equilibrated with 0.01M acetate buffer (pH5.0), followed by collecting fractions which contained a conjugate of the polypeptide and elsinan. Thereafter the fractions were sterilely filtered, concentrated, lyophilized, pulverized in usual manner and admixed with "Finetose", an anhydrous crystalline alpha-maltose commercialized by Hayashibara Co., Ltd., Okayama, Japan, to homogeneity and the mixture was prepared in usual manner into tablets, 200mg each, which contained 100ng of the polypeptide.

The product, which is superior in administrability and stability, is useful as lozenge to treat and prevent pollen allergy.

### Example B-5

### Diagnostic agent

One hundred milligrams of a purified polypeptide obtained by the method in Example A-1(b) was dissolved in 20ml physiological saline, sterilely filtered in usual manner, distributed in 1ml aliquots in sterile vials, lyophilized and sealed to obtain a diagnostic agent.

The product is dissolved in 1ml distilled water for injection and diluted by 10 times in the same distilled water, prior to use in diagnosis of pollen allergy by scratch and intradermal tests.

### Example B-6

### Diagnostic agent

One milligrams of a purified polypeptide obtained by the method in Example A-1(b) was dissolved in 20ml of 50% (v/v) aqueous glycerine solution containing 5% (w/v) sodium chloride, sterilely filtered and distributed in 1ml aliquots in sterile vials in usual manner.

The product is diluted by 20 times in 50% (v/v) aqueous glycerine solution, prior to use in diagnosis of pollen allergy by scratch and intradermal tests.

Several Experiments will be given hereinafter to illustrate effects of the desensitizer according to this invention.

### Experiment 4

### Animal test

In this Experiment laboratory animals were actually administered with conjugates of the polypeptide and specific saccharides according to this invention in order to prove that such a conjugate was efficacious in treatment and prevention of pollen allergy.

### Experiment 4-1

### Prophylactic effect

A group of six 10-12 week-old female BALB/c mice were intraperitoneally administered once every week over 3 weeks with 1 micrograms/mouse of a desensitizer obtained by the method in Example B-1 in terms of polypeptide amount. One week after the last administration, the mice were injected through the same rout with 0.2ml physiological saline containing 1 microgram of a purified polypeptide as antigen obtained by the method in Example A-1(b) and 4mg aluminum hydroxide as adjuvant. Immediately before and one week after administration of the antigen, the mice were bleeded and the blood specimens were determined for immunoglobulin E, G and M antibodies which were specific to the polypeptide.

Separately, an additional system where a mixture containing a purified polypeptide obtained by the method in Example A-1(b) and a purified pullulan, averaged molecular weight of about 200,000 daltons, at a ratio of 1:1 by weight was administered in place of desensitizers was provided as control and treated similarly as above. Amounts of immunoglobulin E antibody specific to the polypeptide were determined by the PCA reaction reported in I. Mota et al., Life Sciences, Vol.8, No.16, Part II, pp.813-820 (1969), while amounts of immunoglobulin G and M antibodies both specific to the polypeptide were determined by the enzyme-linked immunosorbent method reported in S. Yoshitake et al., The Journal of Biochemistry, Vol.92, No.5, pp.1,413-1,424 (1982): All the amounts were represented by averaged antibody titer for 6 mice. The results were as shown Table 21.

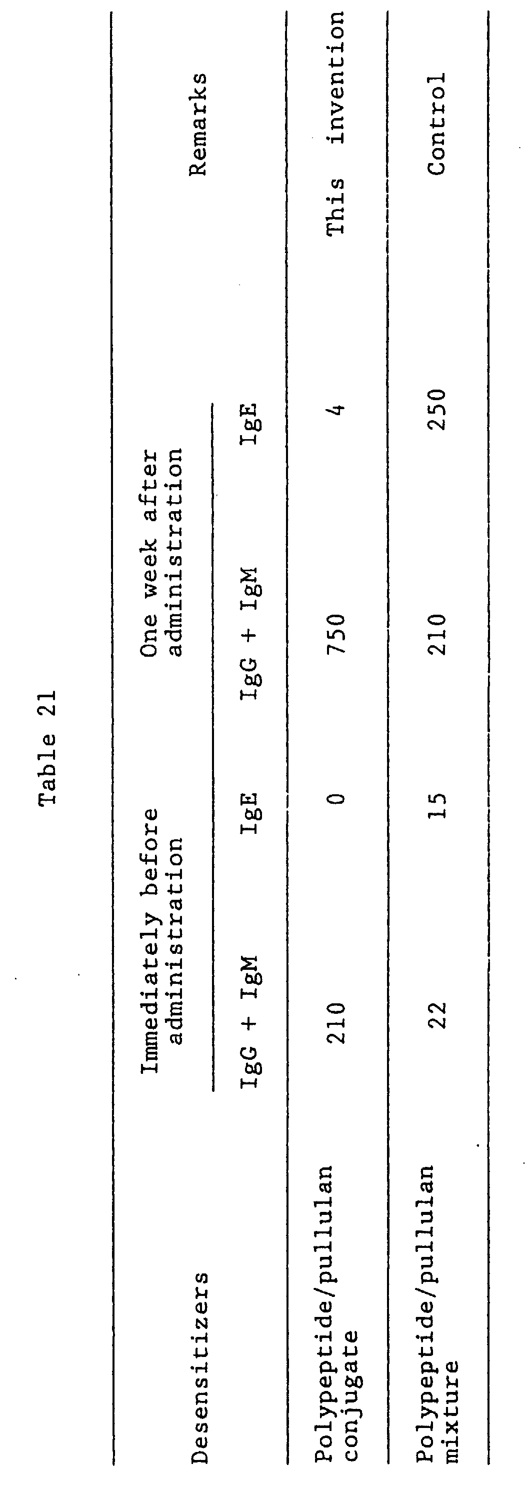

As evident from the results in Table 21, in comparison with control, in the system where the desensitizer of this invention containing a conjugate of the polypeptide and pullulan was previously administered, immunoglobulin G and M antibodies which were effective in desensitization were produced in a notably high level, while the production of immunoglobulin E antibody was suppressed to substantially nondetectable level. Since immunoglobulin E antibody is said to be one of the major causes of undesirable side effects including anaphylactic shock, the fact that administration of the desensitizer of this invention in mice led to a notable suppression of immunoglobulin E antibody would suggest that desensitizers containing conjugates according to this invention are useful in safe and effective prevention of pollen allergy in mammals including human.

### Experiment 4-2

### Therapeutic effect on parenteral administration

A group of six 10-12 week-old female BALB/c mice were intraperitoneally injected one every week over 3 weeks with 0.2ml physiological saline containing one micrograms of a purified polypeptide as antigen obtained by the method in Example A-1(b) and 1 microgram aluminum hydroxide as adjuvant to arise pollen allergy in the mice. One week after the last administration of antigen, the mice were further injected once every week over 3 weeks through the same rout with 100ng/mouse of a desensitizer obtained by the method in Example B-1 in terms of polypeptide amount. One week after the last desensitization, the mice were administered similarly as above only with a fresh preparation of the same antigen so as to induce the production of immunoglobulin E antibody. Immediately before the first administration of desensitizer, one week after the last administration of desensitizer and one week after the reinduction of immunoglobulin E antibody, the mice were bleeded and the blood specimens were determined similarly as in Experiment 4-1 for immunoglobulin E, G and M antibodies specific to the polypeptide.

Separately, an additional system where a mixture containing a purified polypeptide obtained by the method in Example A-1(b) and a purified pullulan, averaged molecular weight of about 200,000 daltons, at a ratio of 1:15 by weight was administered in place of desensitizers was provided as control and treated similarly as above. The results were as shown in Table 22.

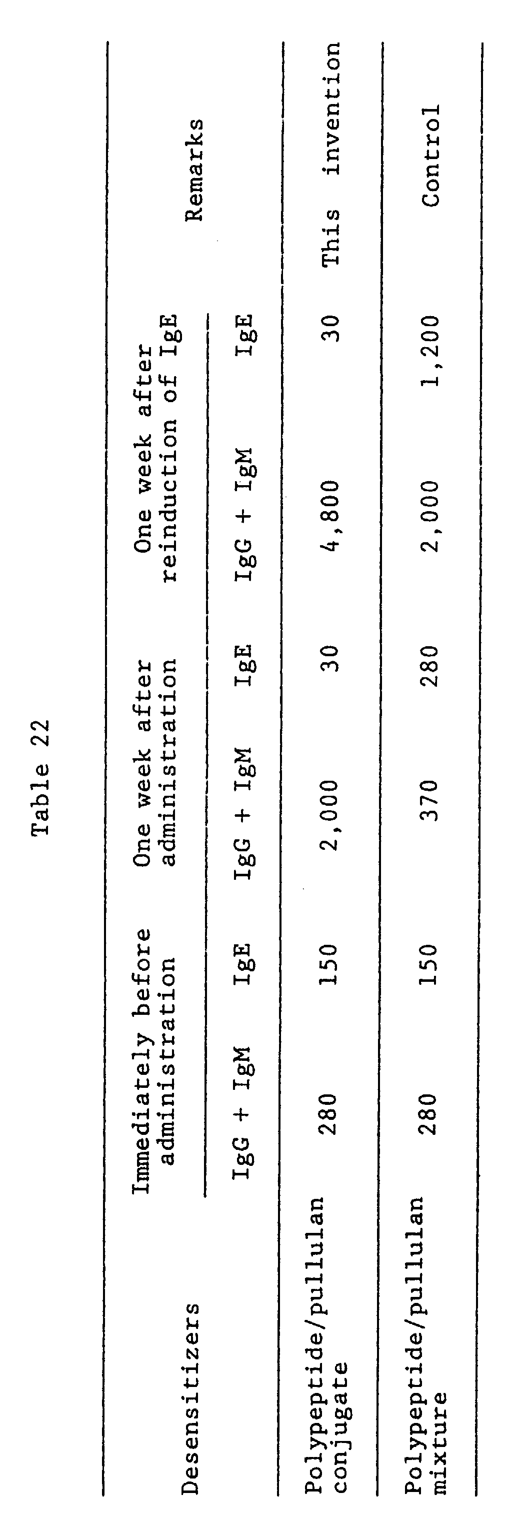

As evident from the results in Table 22, in comparison with control, in the system where a desensitizer containing a conjugate of the polypeptide and pullulan according to this invention was previously administered, high levels of immunoglobulin G and M antibodies were detected even after administration of the desensitizer and also after reinduction of immunoglobulin E antibody. While the production of immunoglobulin E antibody was suppressed to substantially nondetectable level even after reinduction of immunoglobulin E antibody, as well as after administration of the desensitizer. These results would suggest that desensitizers containing conjugates according to this invention are feasible in safe and effective treatment of pollen allergy in mammals including human through parenteral administration.

### Experiment 4-3

### Therapeutic effect on oral administration

A group of six 10-12 week-old female BALB/c mice were intraperitoneally injected once every week over 3 weeks with 0.2ml physiological saline containing 1 microgram of a purified polypeptide as antigen obtained by the method in Example A-1(b) and 4mg aluminum hydroxide as adjuvant to arise pollen allergy in the mice. One week after the last administration of antigen, the mice were orally administrated once every week over 3 weeks with 10 micrograms/mouse of a lozenge, which had been obtained by the method in Example B-4 and then pulverized, in terms of polypeptide amount. One week after the last administration of lozenge, the mice were bleeded and the blood specimens were determined for immunoglobulin A, G and E antibodies.

Separately, an additional system where a solid mixture containing a purified polypeptide obtained by the method in Example A-1(b) and a purified lipopolysaccharide from Salmonella at a ratio of 1:15 by weight was provided as control and treated similarly as above. Amounts of immunoglobulin A and G antibodies both specific to the polypeptide were determined by EIA method reported in R. Meorini et al., Journal of Immunological Methods, Vol.6, pp.355-362 (1975), while amounts of immunoglobulin E antibody specific to the polypeptide was determined similarly as in Experiment 4-1: The amounts were represented by averaged antibody titer for 6 mice. The results were as shown in Table 23.

As evident from the results in Table 23, in comparison with control, in the system where a desensitizer containing a conjugate of the polypeptide and lipopolysaccharide according to this invention was administered, immunoglobulin A and M antibodies were produced in a notably high level, while the production of immunoglobulin E antibody was suppressed to substantially nondetectable level. These would suggest that desensitizers containing conjugates according to this invention are also feasible in safe and effective treatment of pollen allergy in mammals including human even through oral administration.

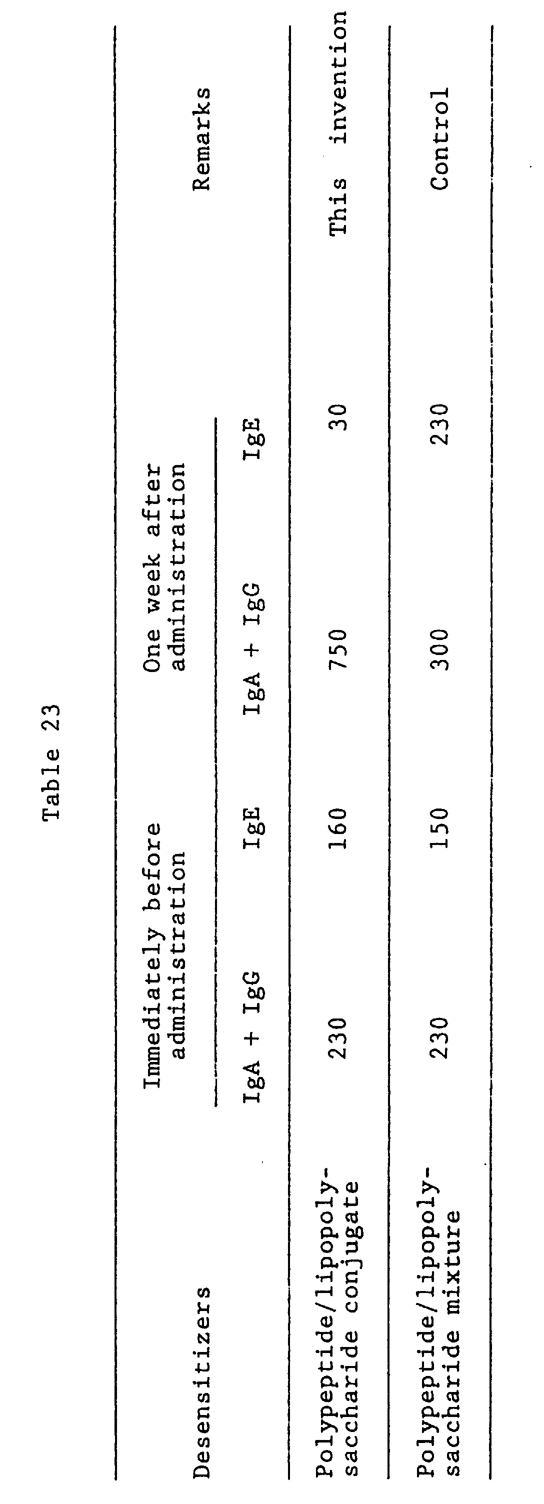

Further, desensitizers obtained by the methods in Examples B-1 through B-4 were injected through intradermal, subcutaneous, intramuscular or intraperitoneal rout or administered in collyrium, collunarium or intranasal nebula form in mice, rats and guinea pigs in accordance with methods which were common in the art (data not shown): As the result, all the desensitizers as tested exhibited significant therapeutic and prophylactic efficacies on pollen allergy without causing serious side effects. These were especially notable in conjugates of the polypeptide and polysaccharides such as pullulan and elsinan which consisted essentially of repeating maltotriose units and in this case doses and administration period needed for prescribed desensitization were extremely reduced or shortened in comparison with case of using other types of conjugates.

### Experiment 4-4

### Acute toxicity

In usual manner 20 day-old mice were orally or intraperitoneally administered with either therapeutic and prophylactic or diagnostic desensitizers obtained by the methods in Examples B-1 through B-4. As the result, it was revealed that the desensitizers beared LD50 of 1,000,000ng/kg or higher for either administration rout. This would suggest that desensitizers containing conjugates of the polypeptide and specific saccharides according to this invention can be used in medicinal agents which are administered in mammals including human.

As described above, the polypeptide of this invention is a novel substance causative of pollen allergy. Since the polypeptide of this invention bears a property of eliciting pollen allergy in mammals including human, it would have extensive uses in desensitizers directed to diagnosis, treatment or prevention of pollen allergy, as well as in diagnosis of pollen allergy by enzyme immunoassay and radioimmuno assays and also in scientific researches to reveal the incident mechanisms of allergic diseases in general. Conjugates of the polypeptide of this invention and specific saccharides have properties of producing in high level immunoglobulin antibodies effective in desensitization but substantially not producing immunoglobulin E antibody which is one of the major causes of undesirable side effects including anaphylactic shock when administered in mammals including human. Because of these properties, in the treatment and prevention of pollen allergy using desensitizers according to this invention, dose and administration period needed for treatment and prevention can be significantly reduced or shortened. The polypeptide useful as such can be easily produced in desired amount with the process of this invention where recombinant DNA technology is applied.

This invention, which exhibits such notable function and effects, would be a significant invention which would make a great contribution in the art.

### SEQUENCE LISTING

(1) GENERAL INFORMATION:
   (i) APPLICANT:
      (A) NAME: KABUSHIKI KAISHA HAYASHIBARA SEIBUTSU KAGAKU KENKYUJO
      (B) STREET: 2-3, 1-CHOME, SHIMOISHII
      (C) CITY: OKAYAMA
      (E) COUNTRY: JAPAN
      (F) POSTAL CODE: 700
   (ii) TITLE OF INVENTION: NOVEL POLYPEPTIDE AND DNA CODING THE SAME
   (iii) NUMBER OF SEQUENCES: 3
   (iv) COMPUTER READABLE FORM:
      (A) MEDIUM TYPE:
      (B) COMPUTER:
      (C) OPERATING SYSTEM:
      (D) SOFTWARE:
   (v) CURRENT APPLICATION DATA:
      APPLICATION NUMBER:
   (vi) PRIOR APPLICATION DATA:
      (A) APPLICATION NUMBERS: JP 299151/1993, JP 344596/1993, JP 346814/1993
      (B) FILING DATES: 5-NOV-1993, 20-DEC-1993, 27-DEC-1993
(2) INFORMATION FOR SEQ ID NO: 1:
   (i) SEQUENCE CHARACTERISTICS:
      (A) LENGTH: 514 amino acids
      (B) TYPE: amino acid
      (D) TOPOLOGY: linear
   (xi) SEQUENCE DESCRIPTION: SEQ ID NO: 1:
   Met Ala Met Lys Phe Ile Ala Pro Met Ala Phe Val Ala Met Gln Leu
    1               5                  10                  15
   Ile Ile Met Ala Ala Ala Glu Asp Gln Ser Ala Gln Ile Met Leu Asp
               20                  25                  30
   Ser Asp Ile Glu Gln Tyr Leu Arg Ser Asn Arg Ser Leu Arg Lys Val
           35                  40                  45
   Glu His Ser Arg His Asp Ala Ile Asn Ile Phe Asn Val Glu Lys Tyr
       50                  55                  60
   Gly Ala Val Gly Asp Gly Lys His Asp Cys Thr Glu Ala Phe Ser Thr
   65                  70                  75                  80
   Ala Trp Gln Ala Ala Cys Lys Lys Pro Ser Ala Met Leu Leu Val Pro
                   85                  90                  95
   Gly Asn Lys Lys Phe Val Val Asn Asn Leu Phe Phe Asn Gly Pro Cys
               100                 105                 110
   Gln Pro His Phe Thr Phe Lys Val Asp Gly Ile Ile Ala Ala Tyr Gln
           115                 120                 125
   Asn Pro Ala Ser Trp Lys Asn Asn Arg Ile Trp Leu Gln Phe Ala Lys
   130 135 140
   Leu Thr Gly Phe Thr Leu Met Gly Lys Gly Val Ile Asp Gly Gln Gly
   145 150 155 160
   Lys Gln Trp Trp Ala Gly Gln Cys Lys Trp Val Asn Gly Arg Glu Ile
   165 170 175
   Cys Asn Asp Arg Asp Arg Pro Thr Ala Ile Lys Phe Asp Phe Ser Thr
   180 185 190
   Gly Leu Ile Ile Gln Gly Leu Lys Leu Met Asn Ser Pro Glu Phe His
   195 200 205
   Leu Val Phe Gly Asn Cys Glu Gly Val Lys Ile Ile Gly Ile Ser Ile
   210 215 220
   Thr Ala Pro Arg Asp Ser Pro Asn Thr Asp Gly Ile Asp Ile Phe Ala
   225 230 235 240
   Ser Lys Asn Phe His Leu Gln Lys Asn Thr Ile Gly Thr Gly Asp Asp
   245 250 255
   Cys Val Ala Ile Gly Thr Gly Ser Ser Asn Ile Val Ile Glu Asp Leu
   260 265 270
   Ile Cys Gly Pro Gly His Gly Ile Ser Ile Gly Ser Leu Gly Arg Glu
   275 280 285
   Asn Ser Arg Ala Glu Val Ser Tyr Val His Val Asn Gly Ala Lys Phe
   290 295 300
   Ile Asp Thr Gln Asn Gly Leu Arg Ile Lys Thr Trp Gln Gly Gly Ser
   305 310 315 320
   Gly Met Ala Ser His Ile Ile Tyr Glu Asn Val Glu Met Ile Asn Ser
   325 330 335
   Glu Asn Pro Ile Leu Ile Asn Gln Phe Tyr Cys Thr Ser Ala Ser Ala
   340 345 350
   Cys Gln Asn Gln Arg Ser Ala Val Gln Ile Gln Asp Val Thr Tyr Lys
   355 360 365
   Asn Ile Arg Gly Thr Ser Ala Thr Ala Ala Ala Ile Gln Leu Lys Cys
       370                 375                 380
   Ser Asp Ser Met Pro Cys Lys Asp Ile Lys Leu Ser Asp Ile Ser Leu
   385                 390                 395                 400
   Lys Leu Thr Ser Gly Lys Ile Ala Ser Cys Leu Asn Asp Asn Ala Asn
                   405                 410                 415
   Gly Tyr Phe Ser Gly His Val Ile Pro Ala Cys Lys Asn Leu Ser Pro
               420                 425                 430
   Ser Ala Lys Arg Lys Glu Ser Lys Ser His Lys His Pro Lys Thr Val
           435                 440                 445
   Met Val Lys Asn Met Gly Ala Tyr Asp Lys Gly Asn Arg Thr Arg Ile
       450                 455                 460
   Leu Leu Gly Ser Arg Pro Pro Asn Cys Thr Asn Lys Cys His Gly Cys
   465                 470                 475                 480
   Ser Pro Cys Lys Ala Lys Leu Val Ile Val His Arg Ile Met Pro Gln
                   485                 490                 495
   Glu Tyr Tyr Pro Gln Arg Trp Met Cys Ser Arg His Gly Lys Ile Tyr
               500                 505                 510
   His Pro
(2) INFORMATION FOR SEQ ID NO: 2:
   (i) SEQUENCE CHARACTERISTICS:
      (A) LENGTH: 1545 base pairs
      (B) TYPE: nucleic acid
      (D) TOPOLOGY: linear
   (xi) SEQUENCE DESCRIPTION: SEQ ID NO: 2:
   ATGGCCATGA AATTCATTGC TCCAATGGCC TTTGTGGCCA TGCAATTGAT TATAATGGCG     60
   GCAGCAGAAG ATCAATCTGC CCAAATTATG TTGGACAGTG ATATCGAACA ATATCTTAGA    120
   TCGAATCGGA GTTTAAGAAA AGTTGAGCAT TCTCGTCATG ATGCTATCAA CATCTTCAAT    180
   GTGGAAAAAT ATGGCGCAGT AGGCGATGGA AAGCATGATT GCACTGAGGC ATTTTCAACA    240
   GCATGGCAAG CTGCATGCAA AAAGCCATCA GCAATGTTGC TTGTGCCAGG CAACAAGAAA    300
   TTTGTTGTAA ACAATTTGTT CTTCAATGGG CCATGTCAAC CTCACTTTAC TTTTAAGGTA    360
   GATGGGATAA TAGCTGCGTA CCAAAATCCA GCTAGCTGGA AGAATAATAG AATATGGTTG    420
   CAGTTTGCTA AACTTACAGG TTTTACTCTA ATGGGTAAAG GTGTAATTGA TGGGCAAGGA    480
   AAACAATGGT GGGCTGGCCA ATGTAAATGG GTCAATGGAC GAGAAATTTG CAACGATCGT    540
   GATAGACCAA CAGCCATTAA ATTCGATTTT TCCACGGGTC TGATAATCCA AGGACTGAAA    600
   CTAATGAACA GCCCCGAATT TCATTTAGTT TTTGGGAATT GTGAGGGAGT AAAAATCATC    660
   GGCATTAGTA TTACGGCACC GAGAGACAGT CCTAACACTG ATGGAATTGA TATCTTTGCA    720
   TCTAAAAACT TTCACTTACA AAAGAACACG ATAGGAACAG GGGATGACTG CGTCGCTATA    780
   GGCACAGGGT CTTCTAATAT TGTGATTGAG GATCTGATTT GCGGTCCAGG CCATGGAATA    840
   AGTATAGGAA GTCTTGGGAG GGAAAACTCT AGAGCAGAGG TTTCATACGT GCACGTAAAT    900
   GGGGCTAAAT TCATAGACAC ACAAAATGGA TTAAGAATCA AAACATGGCA GGGTGGTTCA    960
   GGCATGGCAA GCCATATAAT TTATGAGAAT GTTGAAATGA TAAATTCGGA GAACCCCATA   1020
   TTAATAAATC AATTCTACTG CACTTCGGCT TCTGCTTGCC AAAACCAGAG GTCTGCGGTT   1080
   CAAATCCAAG ATGTGACATA CAAGAACATA CGTGGGACAT CAGCAACAGC AGCAGCAATT   1140
   CAACTTAAGT GCAGTGACAG TATGCCCTGC AAAGATATAA AGCTAAGTGA TATATCTTTG   1200
   AAGCTTACCT CAGGGAAAAT TGCTTCCTGC CTTAATGATA ATGCAAATGG ATATTTCAGT   1260
   GGACACGTCA TCCCTGCATG CAAGAATTTA AGTCCAAGTG CTAAGCGAAA AGAATCTAAA   1320
   TCCCATAAAC ACCCAAAAAC TGTCATGGTT AAAAATATGG GAGCATATGA CAAGGGTAAC   1380
   AGAACACGCA TATTGTTGGG GTCGAGGCCT CCGAATTGTA CAAACAAATG TCATGGTTGC   1440
   AGTCCATGTA AGGCCAAGTT AGTTATTGTT CATCGTATTA TGCCGCAGGA GTATTATCCT   1500
   CAGAGGTGGA TGTGCAGCCG TCATGGCAAA ATCTACCATC CATAA                   1545
(2) INFORMATION FOR SEQ ID NO: 3:
   (i) SEQUENCE CHARACTERISTICS:
      (A) LENGTH: 1545 base pairs
      (B) TYPE: nucleic acid
      (D) TOPOLOGY: linear
   (xi) SEQUENCE DESCRIPTION: SEQ ID NO: 3:

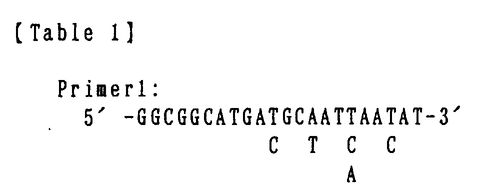

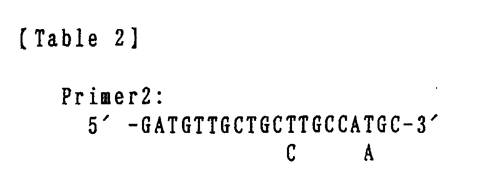

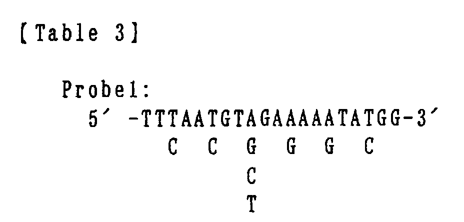

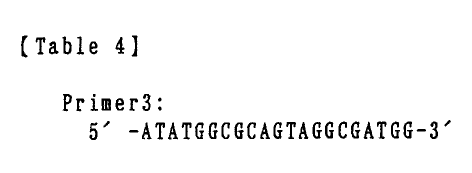

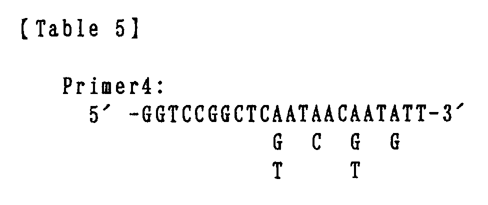

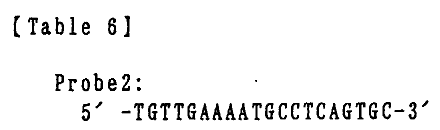

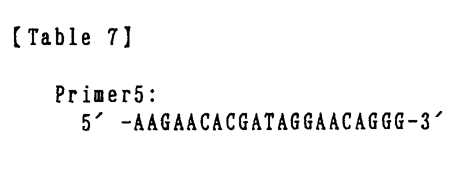

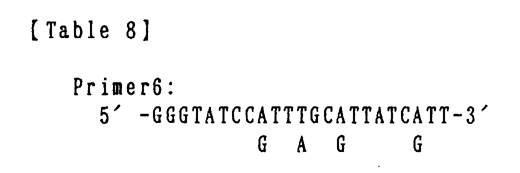

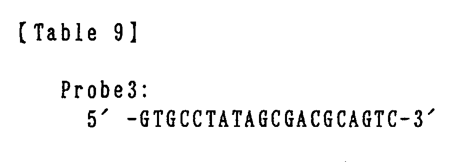

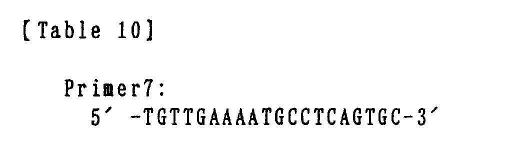

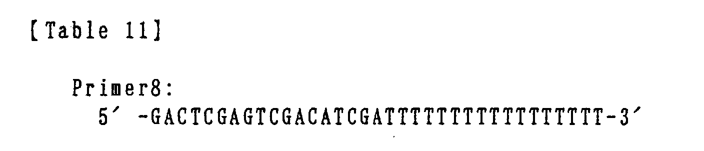

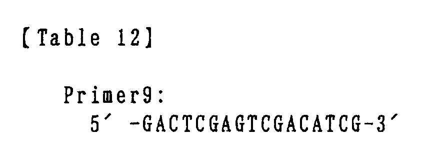

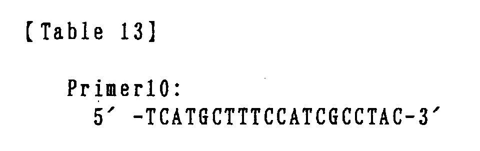

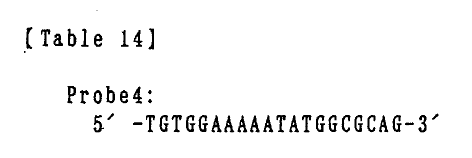

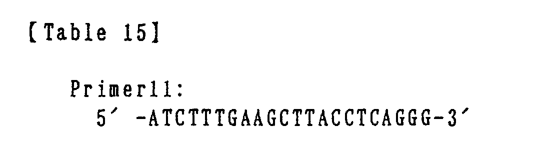

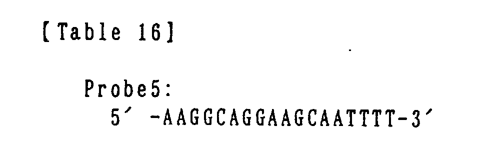

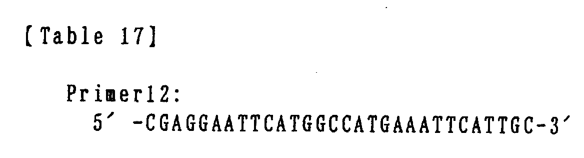

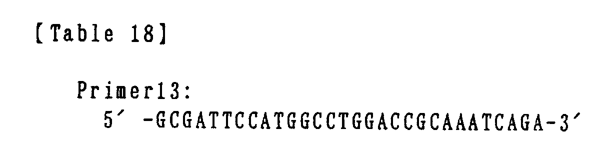

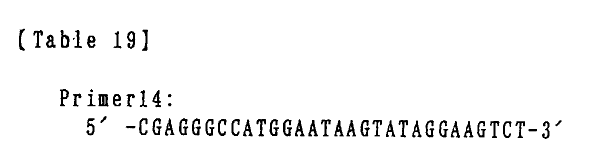

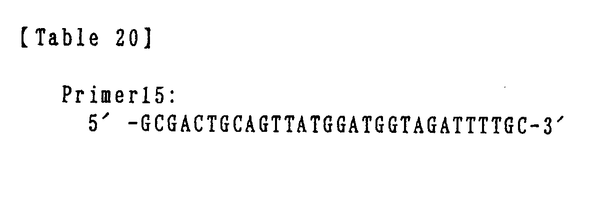

## Claims

1. A polypeptide, which bears the following physicochemical properties:
(1) Molecular weight
55,000±5,000 daltons as determined by SDS-polyacrylamide gel electrophoresis
(2) Isoelectric point
9.0±0.5 as determined by isoelectric point electrophoresis
(3) N-Terminal amino acid sequence
Bearing at N-terminal an amino acid sequence as represented by Ala-Met-Lys-Phe-Ile-Ala-Pro-Met-Ala-Phe-Val-Ala-Met-Gln-Leu-Ile-Ile-Met-Ala-.
(4) Ultraviolet absorption spectrum
Showing an absorption maximum around 280nm.
(5) Solubility in solvents
Soluble in water, physiological saline and phosphate buffer.
(6) Biological activity
Combining with immunoglobulin E antibodies collected from bloods of patients with pollen allergy.
Causative of pollen allergy.
(7) Stability
Inactivated when incubated in aqueous solution (pH7.2) at 100°C for 10 minutes.
Substantially not inactivated even when allowed to stand in aqueous solution (pH7.2) at 4°C for 1 month.

2. The polypeptide according to claim 1 comprising an amino acid sequence as shown in SEQ ID. No. 1 or a homologous sequence to SEQ ID. No. 1 and having an N-terminal sequence comprising the N-terminal sequence of claim 1.

3. The polypeptide of claim 1, which originates from cedar pollen.

4. A DNA, which codes the polypeptide of claim 1.

5. The DNA of claim 4, wherein said polypeptide comprising an amino acid sequence as shown in SEQ ID. No. 1 or a homologous sequence to SEQ ID. No. 1 and having an N-terminal sequence comprising the N-terminal sequence of claim 1.

6. The DNA of claim 4, which bears either a nucleic acid sequence starting at 5'-terminal as shown in the Sequence Listing with SEQ ID No.2 or a nucleic acid sequence which is complementary therewith.

7. The DNA of claim 4, wherein based on degeneracy in genetic codes, one or more nucleic acids in the nucleic acid sequence as shown in the Sequence Listing with SEQ ID No.2 are replaced with other nucleic acids without changing the amino acid sequence as shown in the Sequence Listing with SEQ ID No.1.

8. The DNA of claim 4, which originates from either pollen or male flower of cedar.

9. A replicable recombinant DNA, which comprises the DNA of claim 4, and an expression vector.

10. The replicable recombinant DNA of claim 9, wherein said DNA bears a nucleic acid sequence starting 5'-terminal as shown in the Sequence Listing with SEQ ID No.2 or a nucleic acid sequence which is complementary therewith.

11. The replicable recombinant DNA of claim 9, wherein in said DNA, based on degeneracy of genetic codes, one or more nucleic acids in the nucleic acid sequence in the Sequence Listing with SEQ ID No.2 are replaced with other nucleic acids without changing the amino acid sequence in the Sequence Listing with SEQ ID No.1.

12. The replicable recombinant DNA of claim 9, wherein said DNA originates from pollen or male flower of cedar.

13. The replicable recombinant DNA of claim 9, wherein said expression vector is pKK223-3.

14. A transformant, which comprises the replicable recombinant DNA of claim 9, and an appropriate host to which said replicable recombinant DNA is introduceable.

15. The transformant of claim 14, wherein said DNA bears a nucleic acid sequence starting 5'-terminal as shown in the Sequence Listing with SEQ ID No.2 or a nucleic acid sequence which is complementary therewith.

16. The transformant of claim 14, wherein in said DNA, based on degeneracy of genetic codes, one or more nucleic acids in the nucleic acid sequence as shown in the Sequence Listing with SEQ ID No.2 are replaced with other nucleic acids without changing the amino acid sequence as shown in the Sequence Listing with SEQ ID No.1.

17. The transformant of claim 14, wherein said expression vector is pKK223-3.

18. The transformant of claim 14, wherein said host is a microorganism of Escherichia coli.

19. A process to produce polypeptide, which comprises cultivating in a culture medium the transformant of claim 14, and collecting the produced polypeptide from the resultant culture.

20. The process of claim 19, wherein said polypeptide bears an amino acid sequence as shown in the Sequence Listing with SEQ ID No.1 or an amino acid sequence which is homologous therewith.

21. The process of claim 19, wherein said DNA bears a nucleic acid sequence starting at 5'-terminal as shown in the Sequence Listing with SEQ ID No.2 or a nucleic acid sequence which is complementary therewith.

22. The process of claim 19, wherein in said DNA, based on degeneracy of genetic codes, one or more nucleic acids as shown in the Sequence Listing with SEQ ID No.2 are replaced with other nucleic acids without changing the amino acid sequence as shown in the Sequence Listing with SEQ ID No.1.

23. The process of claim 19, wherein said DNA originates from pollen or male flower of cedar.

24. The process of claim 19, wherein a host in said transformant is a microorganism.

25. The process of claim 19, wherein a vector in said transformant is pKK223-3.

26. The process of claim 19, wherein the produced polypeptide is collected from the resultant culture by one or more members selected from the group consisting of salting out, dialysis, filtration, concentration, centrifugation, gel filtration chromatography, ion exchange chromatography, affinity chromatography, gel electrophoresis and isoelectric point electrophoresis.

27. A desensitizer, which contains as effective ingredient the polypeptide of claim 1.

28. The desensitizer of claim 27, wherein said polypeptide bears an amino acid sequence as shown in the Sequence Listing with SEQ ID No.1 or an amino acid sequence which is homologous therewith and having an N-terminal sequence comprising the N-terminal sequence of claim 1.

29. The desensitizer of claim 27, wherein said polypeptide is collected from a culture which is obtainable by cultivating in a culture medium a transformant which contains a DNA coding the polypeptide described in claim 1.

30. The desensitizer of claim 27, wherein said polypeptide is covalently bound to a saccharide.

31. The desensitizer of claim 30, wherein said saccharide consists essentially of repeating maltotriose units.

32. The desensitizer of claim 27, which contains serum albumin and/or gelatin as stabilizer.

## Patentansprüche

1. Polypeptid mit folgenden physikalisch-chemischen Eigenschaften:
(1) Molekulargewicht
55.000 ± 5.000 Dalton, bestimmt nach der SDS-Polyacrylamid-Gelelektrophorese
(2) Isoelektrischer Punkt
9,0 ± 0,5, bestimmt nach der Elektrophorese für den isoelektrischen Punkt
(3) N-terminale Aminosäuresequenz
enthält am N-Terminus eine Aminosäuresequenz, die durch dargestellt ist
(4) Ultraviolett-Absorptionsspektrum
zeigt ein Absorptionsmaximum um 280 nm
(5) Löslichkeit in Lösungsmitteln
Löslich in Wasser, physiologischer Salzlösung und Phosphatpuffer
(6) Biologische Aktivität
vereint sich mit Immunglobulin-E-Antikörpern, die aus Blut von Patienten mit Pollenallergie gesammelt werden.
Kausativ für Pollenallergie.
(7) Stabilität
Inaktivierung bei Inkubation in wässriger Lösung (pH 7,2) bei 100°C für 10 Minuten.
Im wesentlichen keine Inaktivierung beim Stehenlassen in wässriger Lösung (pH 7,2) bei 4°C für 1 Monat.

2. Polypeptid nach Anspruch 1, welches eine Aminosäuresequenz, wie in SEQ ID Nr. 1 gezeigt oder eine Sequenz, die zur SEQ ID Nr. 1 homolog ist, umfasst und eine N-terminale Sequenz, die die N-terminale Sequenz von Anspruch 1 umfasst, aufweist.

3. Polypeptid nach Anspruch 1, welches aus Zedernpollen stammt.

4. DNA, die für das Polypeptid von Anspruch 1 kodiert.

5. DNA, von Anspruch 4, worin das Polypeptid, das eine Aminosäuresequenz, wie in SEQ ID Nr. 1 gezeigt, oder eine Sequenz, die homolog zur SEQ ID Nr. 1 ist, umfasst und eine N-terminale Sequenz aufweist, die die N-terminale Sequenz von Anspruch 1 umfasst.

6. DNA nach Anspruch 4, die entweder eine Nukleinsäuresequenz, die am 5'-Terminus beginnt, wie im Sequenzprotokoll in der SEQ ID Nr. 2 gezeigt ist oder eine Nukleinsäuresequenz, die damit komplementär ist, enthält.

7. DNA von Anspruch 4, worin, auf der Basis der Degeneration des genetischen Codes, eine oder mehr Nukleinsäuren in der Nukleinsäuresequenz, die im Sequenzprotokoll in der SEQ ID Nr. 2 gezeigt ist, durch andere Nukleinsäuren ersetzt ist bzw. sind, ohne dass die Aminosäuresequenz, die im Sequenzprotokoll in der SEQ ID Nr. 1 gezeigt ist, verändert wird.

8. DNA von Anspruch 4, die entweder von Pollen oder der männlichen Blüte der Zeder stammt.

9. Replizierbare rekombinante DNA, die die DNA von Anspruch 4 und einen Expressionsvektor enthält.

10. Replizierbare rekombinante DNA von Anspruch 9, worin die DNA eine Nukleinsäuresequenz, die am 5'-Terminus, wie im Sequenzprotokoll in der SEQ ID Nr. 2 gezeigt ist, beginnt oder eine Nukleinsäuresequenz, die damit komplementär ist, enthält.

11. Replizierbare rekombinante DNA von Anspruch 9, worin in der DNA, auf der Grundlage der Degeneration des genetischen Codes, eine oder mehr Nukleinsäuren in der Nukleinsäuresequenz im Sequenzprotokoll in der SEQ ID Nr. 2 mit anderen Nukleinsäuren ersetzt ist bzw. sind, ohne dass die Aminosäuresequenz im Sequenzprotokoll in der SEQ ID Nr. 1 verändert wird.

12. Replizierbare rekombinante DNA von Anspruch 9, worin die DNA von Pollen oder der männlichen Blüte der Zeder stammt.

13. Replizierbare rekombinante DNA von Anspruch 9, worin der Expressionsvektor pKK223-3 ist.

14. Transformante, die die replizierbare rekombinante DNA von Anspruch 9 und einen geeigneten Wirt umfasst, in den die replizierbare rekombinante DNA einschleusbar ist.

15. Transformante von Anspruch 14, worin die DNA eine Aminosäuresequenz, die am 5'-Terminus beginnt, wie im Sequenzprotokoll in der SEQ ID Nr. 2 gezeigt ist, oder eine Nukleinsäuresequenz, die damit komplementär ist, enthält.

16. Transformante von Anspruch 14, worin in der DNA, auf der Grundlage der Degeneration des genetischen Codes, eine oder mehr Nukleinsäuren in der Nukleinsäuresequenz, wie im Sequenzprotokoll in der SEQ ID Nr. 2 gezeigt ist, mit anderen Nukleinsäuren ersetzt ist bzw. sind, ohne dass die Aminosäuresequenz, die im Sequenzprotokoll in der SEQ ID Nr. 1 gezeigt ist, verändert wird.

17. Transformante von Anspruch 14, worin der Expressionsvektor pKK223-3 ist.

18. Transformante von Anspruch 14, worin der Wirt ein Mikroorganismus von Escherichia coli ist.

19. Verfahren zur Herstellung eines Polypeptids, bei dem in einem Kulturmedium die Transformante von Anspruch 14 bezüchtet wird und das hergestellte Polypeptid aus der erhaltenen Kultur gesammelt wird.

20. Verfahren von Anspruch 19, worin das Polypeptid eine Aminosäuresequenz, wie im Sequenzprotokoll in der SEQ ID Nr. 1 gezeigt ist, oder eine Aminosäuresequenz, die damit homolog ist, enthält.

21. Verfahren nach Anspruch 19, worin die DNA eine Nukleinsäuresequenz, die am 5'-Terminus beginnt, wie im Sequenzprotokoll in der SEQ ID Nr. 2 gezeigt ist, oder eine Nukleinsäuresequenz, die damit komplementär ist, enthält.

22. Verfahren nach Anspruch 19, worin in der DNA, auf der Grundlage der Degeneration des genetischen Codes, eine oder mehr Nukleinsäuren, wie im Sequenzprotokoll in der SEQ ID Nr. 2 gezeigt ist, mit anderen Nukleinsäuren ersetzt ist bzw. sind, ohne dass die Aminosäuresequenz, wie im Sequenzprotokoll in der SEQ ID Nr. 1 gezeigt ist, verändert wird.

23. Verfahren nach Anspruch 19, worin die DNA von Pollen oder der männlichen Blüte der Zeder stammt.

24. Verfahren nach Anspruch 19, worin ein Wirt in der Transformante ein Mikroorganismus ist.

25. Verfahren nach Anspruch 19, worin ein Vektor in der Transformante pKK223-3 ist.

26. Verfahren nach Anspruch 19, worin das hergestellte Polypeptid aus der erhaltenen Kultur nach einem oder mehr Verfahren der Gruppe Aussalzen, Dialyse, Filtration, Einengung, Zentrifugation, Gelfiltrationschromatographie, Ionenaustauschchromatographie, Affinitätschromatographie, Gelelektrophorese oder Elektrophorese für den isoelektrischen Punkt gesammelt wird.

27. Desensibilisator, der als wirksamen Bestandteil das Polypeptid von Anspruch 1 enthält.

28. Desensibilisator nach Anspruch 27, worin das Polypeptid eine Aminosäuresequenz, wie im Sequenzprotokoll in der SEQ ID Nr. 1 gezeigt ist, oder eine Aminosäuresequenz, die damit homolog ist, enthält und eine N-terminale Sequenz aufweist, die die N-terminale Sequenz von Anspruch 1 umfasst.

29. Desensibilisator von Anspruch 27, worin das Polypeptid aus einer Kultur gesammelt ist, die durch Züchten in einem Kulturmedium einer Transformante, die eine DNA enthält, welche für das in Anspruch 1 beschriebene Polypeptid kodiert, enthält, erhältlich ist.

30. Desensibilisator von Anspruch 27, worin das Polypeptid an ein Saccharid kovalent gebunden ist.

31. Desensibilisator gemäss Anspruch 30, worin das Saccharid im wesentlichen aus sich wiederholenden Maltotrioseeinheiten besteht.

32. Desensibilisator nach Anspruch 27, der als Stabilisator Serumalbumin und/oder Gelatine enthält.

## Revendications

1. Polypeptide, qui présente les propriétés physiques ou chimiques suivantes :
(1) Poids moléculaire :
55 000 ± 5000 daltons, déterminé par électrophorèse sur gel de polyacrylamide au dodécyl-sulfate de sodium
(2) Point iso-électrique :
9,0 ± 0,5, déterminé par électrophorèse au point iso-électrique
(3) Séquence d'amino-acides N-terminale :
portant à l'extrémité N-terminale une séquence d'amino-acides représentée par Ala-Met-Lys-Phe-Ile-Ala-Pro-Met-Ala-Phe-Val-Ala-Met-Gln-Leu-Ile-Ile-Met-Ala-.
(4) Spectre d'absorption de rayonnement ultraviolet :
présentant un maximum d'absorption aux environs de 280 nm.
(5) Solubilité dans des solvants :
soluble dans l'eau, le sérum physiologique et un tampon au phosphate.
(6) Activité biologique :
combinaison avec des anticorps du type immunoglobuline E prélevés dans le sang de patients présentant une allergie au pollen ;
responsable d'une allergie au pollen.
(7) Stabilité :
inactivé lors de sa mise en incubation en solution aqueuse (pH 7,2) à 100°C pendant 10 minutes ;
pratiquement pas inactivé même lorsqu'il est laissé au repos en solution aqueuse (pH 7,2) à 4°C pendant 1 mois.

2. Polypeptide suivant la revendication 1, comprenant une séquence d'amino-acides représentée dans la SEQ ID N° 1 ou une séquence homologue à la SEQ ID N° 1 et ayant une séquence N-terminale comprenant la séquence N-terminale suivant la revendication 1.

3. Polypeptide suivant la revendication 1, qui est issu du pollen de cèdre.

4. ADN, qui code pour le polypeptide suivant la revendication 1.

5. ADN suivant la revendication 4, dans lequel le polypeptide comprenant une séquence d'amino-acides représentée dans la SEQ ID N° 1 ou une séquence homologue à la SEQ ID N° 1 et ayant une séquence N-terminale comprenant la séquence N-terminale suivant la revendication 1.

6. ADN suivant la revendication 4, qui porte une séquence d'acide nucléique commençant à l'extrémité 5'-terminale de la manière représentée dans la liste des séquences par la SEQ ID N° 2 ou une séquence d'acide nucléique qui est complémentaire à cette séquence.

7. ADN suivant la revendication 4, dans lequel, sur la base de la dégénérescence du code génétique, un ou plusieurs acides nucléiques dans la séquence d'acide nucléique représentée dans la Liste des Séquences par la SEQ ID N° 2 sont remplacés par d'autres acides nucléiques sans modification de la séquence d'amino-acides de la manière représentée dans la Liste des Séquences par la SEQ ID N° 1.

8. ADN suivant la revendication 4, qui provient du pollen ou d'une fleur mâle de cèdre.

9. ADN recombinant réplicable, qui comprend l'ADN suivant la revendication 4 et un vecteur d'expression.

10. ADN recombinant réplicable suivant la revendication 9, qui porte une séquence d'acide nucléique commençant à l'extrémité 5'-terminale de la manière représentée dans la Liste des Séquences par la SEQ ID N° 2 ou une séquence d'acide nucléique qui est complémentaire avec cette séquence.

11. ADN recombinant réplicable suivant la revendication 9, dans lequel, dans ledit ADN, sur la base de la dégénérescence du code génétique, un ou plusieurs acides nucléiques dans la séquence d'acide nucléique représentée dans la Liste des Séquences par la SEQ ID N° 2 sont remplacés par d'autres acides nucléiques sans modification de la séquence d'amino-acides représentée dans la Liste des Séquences par la SEQ ID N° 1.

12. ADN recombinant réplicable suivant la revendication 9, dans lequel ledit ADN provient du pollen ou d'une fleur mâle de cèdre.

13. ADN recombinant réplicable suivant la revendication 9, dans lequel le vecteur d'expression est le vecteur pKK223-3.

14. Transformant, qui comprend l'ADN recombinant réplicable suivant la revendication 9 et un hôte approprié dans lequel ledit ADN recombinant réplicable peut être introduit.

15. Transformant suivant la revendication 14, dans lequel l'ADN porte une séquence d'acide nucléique commençant à l'extrémité 5'-terminale de la manière représentée dans la Liste des Séquences par la SEQ ID N° 2 ou une séquence d'acide nucléique qui est complémentaire avec cette séquence.

16. Transformant suivant la revendication 14, dans lequel, dans ledit ADN, sur la base de la dégénérescence du code génétique, un ou plusieurs acides nucléiques dans la séquence d'acide nucléique représentée dans la Liste des Séquences par la SEQ ID N° 2 sont remplacés par d'autres acides nucléiques sans modification de la séquence d'amino-acides représentée dans la Liste des Séquences par la SEQ ID N° 1.

17. Transformant suivant la revendication 14, dans lequel le vecteur d'expression est le vecteur pKK223-3.

18. Transformant suivant la revendication 14, dans lequel l'hôte est un micro-organisme appartenant à l'espèce Escherichia coli.

19. Procédé pour la production d'un polypeptide, qui comprend les étapes consistant à cultiver dans un milieu de culture le transformant suivant la revendication 14, et à recueillir le polypeptide produit à partir de la culture résultante.

20. Procédé suivant la revendication 19, dans lequel le polypeptide porte une séquence d'amino-acides représentée dans la Liste des Séquences par la SEQ ID N° 1 ou une séquence d'amino-acides qui est homologue avec cette séquence.

21. Procédé suivant la revendication 19, dans lequel l'ADN porte une séquence d'acide nucléique commençant à l'extrémité 5'-terminale de la manière représentée dans la Liste des Séquences avec la SEQ ID N° 2 ou une séquence d'acide nucléique qui est complémentaire avec cette séquence.

22. Procédé suivant la revendication 19, dans lequel, dans l'ADN, sur la base de la dégénérescence du code génétique, un ou plusieurs acides nucléiques représentés dans la Liste des Séquences par la SEQ ID N° 2 sont remplacés par d'autres acides nucléiques sans modification de la séquence d'amino-acides représentée dans la liste des Séquences avec la SEQ ID N° 1.

23. Procédé suivant la revendication 19, dans lequel l'ADN provient du pollen ou d'une fleur mâle de cèdre.

24. Procédé suivant la revendication 19, dans lequel un hôte dans ledit transformant est un micro-organisme.

25. Procédé suivant la revendication 19, dans lequel un vecteur dans le transformant est le pKK223-3.

26. Procédé suivant la revendication 19, dans lequel le polypeptide produit est recueilli en le séparant de la culture résultante par une ou plusieurs opérations choisies dans le groupe consistant en un relargage, une dialyse, une filtration, une concentration, une centrifugation, une chromatographie de filtration sur gel, une chromatographie d'échange d'ions, une chromatographie d'affinité, une électrophorèse sur gel et une électrophorèse au point iso-électrique.

27. Désensibilisant, qui contient comme ingrédient efficace le polypeptide suivant la revendication 1.

28. Désensibilisant suivant la revendication 27, dans lequel le polypeptide porte une séquence d'amino-acides représentée dans la Liste des Séquences par la SEQ ID N° 1 ou une séquence d'amino-acides qui est homologue avec cette séquence, ayant une séquence N-terminale comprenant la séquence N-terminale suivant la revendication 1.

29. Désensibilisant suivant la revendication 27, dans lequel le polypeptide est recueilli en le séparant d'une culture qui peut être obtenue en cultivant dans un milieu de culture un transformant qui contient un ADN codant pour le polypeptide décrit dans la revendication 1.

30. Désensibilisant suivant la revendication 27, dans lequel le polypeptide est lié par covalence à un saccharide.

31. Désensibilisant suivant la revendication 30, dans lequel le saccharide consiste essentiellement en motifs maltotriose répétés.

32. Désensibilisant suivant la revendication 27, qui contient de la sérum-albumine et/ou de la gélatine comme stabilisant.
